# EUROPÄISCHE PATENTSCHRIFT

(11) **EP 1 813 588 B1**
(45) Veröffentlichungstag und Bekanntmachung des Hinweises auf die Patenterteilung: **04.01.2012**
(21) Anmeldenummer: 06123991.9
(22) Anmeldetag: 14.11.2006
(51) Int. Cl.: C07C 41/06, C07C 43/04

(54) **Verfahren zur Herstellung von Ethyl-tert.-Butylether aus technischen Mischungen von C4-Kohlenwasserstoffen**
Process for the preparation of ethyl tert-butyl ether from C4-hydrocarbon cuts
Procédé de préparation de l'éthyl tert-butyl éther à partir de mélanges d'hydrocarbures C4

(30) Priorität: 28.12.2005 DE 102005062722
(43) Veröffentlichungstag der Anmeldung: 01.08.2007
(73) Patentinhaber: Evonik Oxeno GmbH, 45772 Marl (DE)
(72) Erfinder: Praefke, Jochen, 45739 Oer-Erkenschwick (DE); Rix, Armin, 45770 Marl (DE); Santiago Fernandez, Silvia, 33009 Oviedo (ES); Grömping, Matthias, Kenner, LA 70065 (US); Höper, Frank, 45721 Haltern am See (DE); Peters, Udo, 45770 Marl (DE); Leistner, Jörg, 45721 Haltern am See (DE); Nierlich, Franz, 45768 Marl (DE); Röttger, Dirk, 45657 Recklinghausen (DE)
(74) Vertreter: Hirsch, Hans-Ludwig

(56) Entgegenhaltungen:
- EP-A1- 0 071 032
- WO-A-97/32838
- DE-A1- 2 521 964
- DE-A1- 10 334 001
- US-A- 4 161 496
- US-B1- 6 472 568

## Beschreibung

Die Erfindung betrifft ein Verfahren zur Herstellung von Ethyl-tert.-Butylether (ETBE) aus technischen Mischungen von C₄-Kohlenwasserstoffen, die mindestens 1-Buten, Isobuten, n-Butan und 2-Butene aufweisen.

1-Buten, Isobuten, 2-Butene und deren Folgeprodukte werden in großen Mengen aus technischen C₄-Schnitten, beispielsweise dem C₄-Schnitt aus Steamcrackern oder FCC-Einheiten, gewonnen. Diese Gemische bestehen im Wesentlichen aus Butadien, den Monoolefinen Isobuten, 1-Buten und den beiden 2-Butenen sowie den gesättigten Kohlenwasserstoffen Isobutan und n-Butan. Wegen der geringen Siedepunktsunterschiede der Inhaltsstoffe und deren geringen Trennfaktoren ist eine destillative Aufarbeitung schwierig und nicht wirtschaftlich. Die Gewinnung von linearen Butenen und von anderen Produkten erfolgt daher meistens durch eine Kombination von chemischen Umsetzungen und physikalischen Trennoperationen.

Der erste Schritt, den dabei alle Aufarbeitungsvarianten gemeinsam haben, ist häufig die Entfernung des größten Teils des Butadiens. Kann Butadien gut vermarktet werden oder besteht ein Eigenverbrauch, wird es üblicherweise durch Extraktion oder Extraktivdestillation abgetrennt. Im anderen Fall wird es bis zu einer Restkonzentration von circa 2000 Massen-ppm selektiv zu linearen Butenen hydriert. Zurück bleibt in beiden Fällen ein Kohlenwasserstoffgemisch (sogenanntes Raffinat I oder hydriertes Crack-C₄), das neben den gesättigten Kohlenwasserstoffen n-Butan und Isobutan die Olefine Isobuten, 1-Buten und 2-Butene (cis und trans) enthält.

Aus diesem Gemisch lässt sich durch Umsetzung des enthaltenen Isobutens mit Alkohol der entsprechende Alkyl-tert.-butylether (ATBE), insbesondere Ethyl-tert.-butylether (ETBE) und Methyl-tert.-butylether (MTBE) gewinnen. Nach der Umsetzung des Isobutens und Abtrennung des Alkyl-tert.-butylether verbleibt ein Kohlenwasserstoffgemisch (Raffinat II), das die linearen Butene und die gesättigten Kohlenwasserstoffe Isobutan und n-Butan enthält. Dieses Gemisch kann destillativ weiter aufgetrennt werden, beispielsweise in Isobutan und 1-Buten und ein Gemisch aus den beiden 2-Butenen und n-Butan. Aus der 1-Buten-haltigen Fraktion kann in weiteren Destillationsschritten 1-Buten in hoher Reinheit erhalten werden, welches nur noch geringe Mengen an Isobuten enthält. Dies ist notwendig, da 1-Buten im großen Maße als Comonomer in der Ethylenpolymerisation eingesetzt wird, wo Isobuten-Verunreinigungen unerwünscht sind. Typische Spezifikationen von 1-Buten beschränken daher den Gehalt an Isobuten im 1-Buten auf unter 2000 ppm. Verfahren zur Herstellung von ATBE aus Isobuten aufweisenden C₄-Kohlenwasserstofffraktionen weisen deshalb insbesondere dann eine hohe Wirtschaftlichkeit auf, wenn die Herstellung von ATBE ohne große Verluste an n-Butenen gelingt.

Für die Umsetzung von Isobuten mit Alkoholen, beispielsweise Methanol oder Ethanol, zu den entsprechenden tertiären Butylethern wurden diverse verfahrenstechnische Varianten entwickelt. Die Technik der Reaktivdestillation hat sich dabei als besonders nützlich zur Ereichung hoher Isobuten-Umsätze erwiesen.

Das technisch bedeutendste Verfahren ist die Umsetzung von Isobuten mit Methanol zu Methyl-tert.-butylether (MTBE), das hauptsächlich als Kraftstoffzusatz große Verwendung findet. Auf Grund der immer größeren Verfügbarkeit von Ethanol aus nachwachsenden Rohstoffen erhöht sich auch die Nachfrage nach ETBE als Kraftstoffzusatz.

In EP 0 048 893 wird ein Verfahren zur gleichzeitigen Herstellung von Isobutenoligomeren und Alkyl-tert.-butylether (ATBE) aus C₄-Schnitten in einem Reaktor dargelegt. Als Katalysator wird ein saures Ionenaustauscherharz, das teilweise mit Metallen der siebten und achten Nebengruppe des Periodensystems der Elemente in elementarer Form (Oxidationsstufe 0) belegt ist, verwendet. Die Produkte und die nicht umgesetzten C₄-Kohlenwasserstoffe werden destillativ getrennt. Bei diesem Verfahren gehen circa 8 % der linearen Butene durch Oligomerisierung verloren. Der Verlust an 1-Buten liegt bei 7 %. Der Hauptnachteil dieses Verfahren ist jedoch, dass kein vollständiger Isobutenumsatz erreicht wird, sodass der Isobutengehalt in der abgetrennten C₄-Kohlenwasserstoff-Fraktion zu hoch ist, um daraus ein spezifikationsgerechtes 1-Buten zu gewinnen.

In DE 25 21 964 wird ein zweistufiges Verfahren zur Herstellung von Alkyl-tert.-Butylethern beschrieben, bei dem in einer ersten Stufe Isobuten mit Alkohol umgesetzt wird, der entstandene Ether von Produktgemisch der ersten Stufe abgetrennt wird und der verbleibende Rest des Produktgemisches in eine zweite Reaktionsstufe geführt wird, in welcher verbleibendes Isobuten wiederum mit Alkohol umgesetzt wird.

Auch in US 4,797,133 wird ein Verfahren beschrieben, bei dem in einer ersten Reaktionszone der Ausgangskohlenwasserstoffstrom an Isobuten z. B. durch Reaktion zum Methyl-tert.-Butylether (MTBE) und Abtrennung des entstandenen Ethers verarmt wird und der verbleibende Rest des Ausgangskohlenwasserstoffstroms einer Etherifizierungsstufe zugeführt wird, in welcher das restliche Isobuten umgesetzt wird.

In EP 0 071 032 wird ebenfalls ein zweistufiges Verfahren zur Herstellung von ETBE beschrieben, bei dem zwischen erster und zweiter Stufe das in der ersten Stufe gebildete ETBE aus dem Reaktionsgemisch abgetrennt wird.

Ein weiteres zweistufiges Verfahren zur Herstellung von Alkylethern ist aus US-A-4 161 496 bekannt. Dabei wird in einem ersten Schritt zunächst ein Teil des in den C4-Mischungen enthaltenen Isobutens verethert, anschließend wird der Ether vom restlichen Kohlenwasserstoffgemisch abgetrennt und letzteres in einem zweiten Schritt von restlichem Isobuten befreit.

Nachteilig an all diesen Verfahren sind die großen Mengen, die in den zweiten Reaktionsschritt gefahren werden müssen. Es bestand daher die Aufgabe, ein verbessertes Verfahren zur Herstellung von ETBE als Kraftstoffzusatz bereitzustellen.

### Beschreibung der Erfindung

Überraschenderweise wurde nun gefunden, dass ETBE aus einer technischen Mischung von C₄-Kohlenwasserstoffen, die mindestens 1-Buten, Isobuten, n-Butan und 2-Butene enthalten, in hoher Ausbeute und mit geringerem Aufwand hergestellt werden kann, wenn bei der zweistufigen Umsetzung von Isobuten mit Ethanol zu ETBE nach der ersten Stufe nach der Abtrennung des hergestellten ETBEs der verbleibende Kohlenwasserstoffstrom zunächst destillativ in eine mindestens 2-Butene und n-Butan aufweisende Fraktion und in eine 1-Buten, Isobuten und ggf. Isobutan aufweisende Fraktion getrennt wird, wobei letztere dann in die zweite Veretherungsstufe geführt wird.

Gegenstand der vorliegenden Erfindung ist deshalb ein Verfahren zur Herstellung von Ethyltert.-Butylether (ETBE) aus technischen Mischungen von C₄-Kohlenwasserstoffen I, die mindestens 1-Buten, Isobuten, n-Butan und 2-Butene enthalten, welches gekennzeichnet ist durch die Verfahrensschritte.
a) Umsetzung von Teilen des in der technischen Mischung enthaltenen Isobutens mit Ethanol in Gegenwart eines sauren Katalysators zu ETBE,
b) Abtrennung der nicht umgesetzten C₄-Kohlenwasserstoffe III aus dem Austrag der Stufe a) durch thermische Trennverfahren unter Erhalt einer ETBE aufweisenden Fraktion II,
c) destillative Trennung der C₄-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) erneute Umsetzung des in Fraktion IV enthaltenen Isobutens mit Ethanol VI in Gegenwart eines sauren Katalysators zu ETBE,
e) Abtrennung der nicht umgesetzten C₄-Kohlenwasserstoffe VIII aus dem Austrag von Stufe d) unter Erhalt einer ETBE aufweisenden Fraktion VII, wobei die in Schritt e) erhaltene C₄-Kohlenwasserstofffraktion VIII durch Destillation in einer oder mehreren Destillationskolonnen zu einem 1-Buten, welches einen Gehalt an Isobuten von kleiner 2000 wppm aufweist, aufgearbeitet wird.

Der besondere Vorteil des erfindungsgemäßen Verfahrens besteht darin, dass durch die zusätzliche Abtrennung der 2-Butene und n-Butane aus dem Reaktionsgemisch in Schritt c) ein geringer Völumenstrom durch den Reaktionsschritt d) gefahren werden muss, weshalb der oder die Reaktoren im Verfahrensschritt d) relativ klein ausgeführt sein können oder bei gleicher Größe im Vergleich zu herkömmlichen Verfahren höhere Umsätze erzielt werden können. Ein weiterer Vorteil der Abtrennung der 2-Butene und n-Butane in Schritt c) besteht darin, dass die Eingangskonzentration an Isobuten im Schritt d) entsprechend höher ist, was die Umsetzung des Isobutens im Verfahrensschritt d) vereinfacht.

Ein weiterer Vorteil des erfindungsgemäßen Verfahrens besteht darin, dass durch den verringerten Volumenstrom in den Stufen d) und e) eine deutlich geringere Menge an Energie, z. B. in Form von Wärme, in der Stufe e) eingesetzt werden muss.

Durch die Möglichkeit, Bioethanol einsetzen zu können, wird zudem eine attraktive Rohstoffquelle für die Herstellung von ETBE genutzt, welches das auf fossilen Rohstoffen basierende MTBE ersetzen kann.

Nachfolgend wird das erfindungsgemäße Verfahren beispielhaft beschirieben.

### Verfahrensschritt a)

Vorzugsweise wird der Verfahrensschritt a) so durchgeführt, dass der Umsatz des Isobutens im Verfahrensschritt a) von größer 50 %, vorzugsweise größer 70 %, bevorzugt größer 80 %, besonders bevorzugt größer 90 % und ganz besonders bevorzugt größer 95 % beträgt. Die Höhe des Umsatzes an Isobuten kann z. B. durch die Anzahl der in Schritt a) verwendeten Reaktoren oder durch Wahl von geeigneten Reaktionsbedingungen, die der Fachmann leicht durch einfache Vorversuche ermitteln kann, gesteuert werden.

Die Veretherung des Isobutens wird als sauer katalysierte Reaktion durchgeführt. Als Ethanol kann hochreiner Ethanol, reiner Ethanol oder Ethanol verwendet werden, der geringe Mengen an Verunreinigungen aufweist. Bevorzugt liegt die Reinheit des eingesetzten Ethanols, angegeben in Massen-% an Ethanol, größer 90 %, besonders bevorzugt größer 95 % und ganz besonders bevorzugt gleich oder größer 99 %. Ethanol, welcher eine Reinheit von größer-gleich 99 Massen-% aufweist, kann z. B. Bioethanol sein. Der Gehalt an Wasser liegt bevorzugt unter 3 Massen-%, besonders bevorzugt unter 1 Massen-%, ganz besonders bevorzugt unter 0,5 Massen-%. Der Einsatzalkohol kann durch Azeotrop-Destillation und/oder Membranverfahren getrocknet werden.

Besonders bevorzugt wird als Ethanol vergällter Ethanol eingesetzt. Ganz besonders bevorzugt wird als Ethanol ein Ethanol eingesetzt, welches als Vergällungsmittel ETBE, vorzugsweise in einer Konzentration von 0 bis 5 Massen-%, bevorzugt von 0,005 bis 1 Massen-%, besonders bevorzugt 0,05 bis 1 Massen-% und ganz besonders bevorzugt von 0,01 bis 0,2 Massen-% aufweist. In Deutschland wird besonders bevorzugt Ethanol eingesetzt, welches mindestens von 0,1 bis 1 Massen-% Vergällungsmittel aufweist.

Für die Umsetzung von Isobuten mit Alkoholen, insbesondere mit Methanol zu Methyl-tert.-butylether, wurden diverse Verfahrensvarianten entwickelt (vergleich: Ullmann's Encyclopedia of Industrial Chemistry, Online Version, 2004, Wiley & Sons, Stichwort Methyl Tert-Butyl Ether, und dort zitierte Literatur; Obenaus, Fritz; Droste, Wilhelm, Erdoel & Kohle, Erdgas, Petrochemie (1980), 33(6), 271 bis 275; DE 26 29 769; DE 28 53 769). Prinzipiell sind alle Verfahren zur Umsetzung des Isobutens mit Alkoholen im Rahmen dieser Erfindung als Verfahrensschritt a) geeignet. Bevorzugt werden Verfahren, bei denen die Umsetzung in flüssiger Phase an einem sauren Ionenaustauscherharz erfolgt.

Als Reaktoren, in denen das Ethanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren etc.) eingesetzt werden. Sie können mit oder ohne partieller Rückführung, wobei gegebenenfalls der Rückführungsstrom gekühlt werden kann, betrieben werden.

In einer bevorzugten Ausführungsform wird die Umsetzung des Isobutens mindestens zweistufig durchgeführt, wobei die erste Stufe als adiabatischer Festbettreaktor mit Rückführung (Schlaufenreaktor) und die folgenden Stufen als Festbettstufen ohne Rückführung und/oder als Reaktivdestillation betrieben werden. Das Verhältnis von rückgeführter Menge zu Frischzulauf (C₄-Kohlenwasserstoffe und Ethanol) liegt bevorzugt bei 0,5 bis 20 t/t, besonders bevorzugt bei 1 bis 5 t/t und ganz besonders bevorzugt bei 2 bis 3 t/t. Die Reaktoren können bei Temperaturen von vorzugsweise 10 bis 160 °C, bevorzugt von 30 bis 110°C, betrieben werden. Der Druck in den Festbettstufen beträgt vorzugsweise 5 bis 50 bar_{abs}. (bara), bevorzugt 7,5 bis 20 bara und besonders bevorzugt von 8 bis 13 bara. Der Kreislaufreaktor weist vorzugsweise eine Eingangstemperatur von 35 bis 50 °C und eine Ausgangstemperatur von 50 bis 70 °C auf und wird bevorzugt bei 10 bis 13 bara betrieben. Da das thermodynamische Gleichgewicht zwischen Ethanol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben. Besonders bevorzugt wird im Verfahrensschritt a) ein Reaktorsystem eingesetzt, welches drei in Reihe geschaltete Reaktoren aufweist, von denen der erste Reaktor als Schlaufenreaktor betrieben wird und die beiden nachfolgenden Reaktoren im geraden Durchgang betrieben werden. Es kann vorteilhaft sein, wenn in dem Verfahrensschritt a) mehrere, vorzugsweise zwei dieser Reaktorsysteme vorhanden sind, so dass bei Reparaturarbeiten, wie z. B. Katalysatorwechsel, an einem Reaktor eines des Reaktorsysteme der Verfahrensschritt a) in dem anderen Reaktorsystem ohne Unterbrechung der Verfahrens (allerdings unter Halbierung des Durchsatzes) weiter durchgeführt werden kann. Bei Verwendung von Reaktorsystemen aus drei Reaktoren werden die Reaktoren vorzugsweise bei einer Temperatur von 30 bis 80 °C, bevorzugt 40 bis 75 °C und einem Druck von 5 bis 20 bara, bevorzugt 7 bis 15 bara betrieben, wobei vorzugsweise die Temperatur in den Reaktoren von ersten zum letzten Reaktor abfällt. Die Reaktoren nach dem Kreislaufreaktor weisen vorzugsweise eine Eingangstemperatur von 30 bis 50 °C und eine Ausgangstemperatur von 35 bis 45 °C auf und werden bevorzugt ebenfalls bei 8 bis 13 bara betrieben.

Das molare Verhältnis von Ethanol zu Isobuten beträgt im erfindungsgemäßen Verfahrensschritt a) vorzugsweise von 5 zu 1 bis 0,9 zu 1, bevorzugt von 2 zu 1 bis 1 zu 1 und besonders bevorzugt von 1,2 zu 1 bis 1 zu 1. Da im Verfahrensschritt a) ein geringerer Umsatz an Isobuten akzeptiert werden kann, kann im Vergleich zu Verfahrensschritt d) ein geringerer Ethanolüberschuss vorteilhaft sein.

In einer bevorzugten Ausführungsform wird die Addition des Ethanols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt a) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus dem Isobuten-haltigen technischen Kohlenwasserstoffgemisch I und Ethanol ein Reaktionsgemisch hergestellt, das hinsichtlich seiner Isobuten-, Ethanol- und ETBE-Konzentration in der Nähe des thermodynamischen Gleichgewichts liegt. Der Umsatz des Isobutens beträgt dabei bevorzugt mehr als 90 %. Dieses Gemisch wird in der nächsten/letzten Reaktionsstufe in die Reaktivdestillationskolonne eingespeist, wo ein weiterer Teil des Isobutens zum Ether umgesetzt wird. Besonders bevorzugt erfolgt die Durchführung des Verfahrensschrittes a) in einem Reaktorsystem, welches drei in Reihe geschaltete Reaktoren, vorzugsweise Festbettreaktoren, von denen der erste vorzugsweise in Schlaufenfahrweise und die beiden nachfolgenden Reaktoren im geraden Durchgang betrieben werden, und eine Reaktivdestillation aufweist, wobei der Reaktoraustrag des letzten der in Reihe geschalteten Reaktoren in die Reaktivdestillation geführt wird.

Die Umsetzung des Isobutens mit Ethanol zum entsprechenden tertiären Butylether erfolgt in der Reaktivdestillation in Abhängigkeit vom Druck vorzugsweise im Temperaturbereich von 40 bis 140 °C, bevorzugt von 60 bis 90 °C, besonders bevorzugt von 65 bis 85 °C (Temperatur im Bereich der Kolonne, in der sich der Katalysator befindet. Die Sumpftemperatur der Kolonne kann deutlich höher liegen). Die Reaktivdestillationskolonne wird vorzugsweise bei Drücken, gemessen am Kolonnenkopf, von 3 bara bis 15 bara, bevorzugt 7 bara bis 13 bara betrieben, insbesondere von 8 bara bis 11 bara.

Weist der Verfahrensschritt a) des erfindungsgemäßen Verfahrens eine Reaktivdestillation auf, so wird, wie in DE 101 02 082 für MTBE beschrieben, das Isobuten aufweisende C₄-Kohlenwasserstoffgemisch zusammen mit Ethanol in den ersten der Vorreaktoren eingespeist. Dabei wird der Ethanol bevorzugt im Überschuss eingesetzt. In den Vorreaktoren entsteht ein Gemisch, in dem Isobuten, Ethanol und ETBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Ethanol enthalten sein, als für die vollständige Umsetzung des noch vorhandenen Isobutens gebraucht wird. Der Ethanolüberschuss sollte jedoch derart bemessen werden, dass eine ausreichende Ethanolmenge für das sich bildende Azeotrop aus Ethanol und C₄-Kohlenwasserstoffen vorhanden ist.

Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Optional kann, wenn der Ethanolgehalt im Kolonnenzulauf zur Reaktivdestillationskolonne unter dem maximal zulässigen Wert liegt, zusätzlicher Ethanol zugemischt werden. Darüber hinaus kann am Kopf der Reaktivdestillationskolonne oberhalb des Kolonnenzulaufs unterhalb eines Flüssigkeitsverteilers oder in einem Flüssigkeitsverteiler oberhalb oder im Bereich der Reaktivzone, vorzugsweise im Bereich der Reaktivzone über eine gesonderte Einrichtung eine Ethanol-Einspeisung erfolgen. Eine zusätzliche Einspeisung von Ethanol kann z. B. in den Rücklauf der Kolonne oder direkt in die reaktiven Packungen erfolgen. Die zusätzliche Ethanolzugabe sollte so bemessen sein, dass in den Packungen der Reaktivzone der Ethanolgehalt in der Flüssigphase vorzugsweise größer-gleich 1,5 Massen-%, bevorzugt größer-gleich 2 Massen-% und besonders bevorzugt von 2 bis 3 Massen-% beträgt. Durch die Zugabe von Ethanol in die Reaktionszone wird sichergestellt, dass trotz der Abreaktion ausreichend Ethanol als Edukt zur Verfügung steht.

Bevorzugt weist die Reaktivdestillationskolonne oberhalb der Katalysatorpackung einen Bereich rein destillativer Trennung auf, besonders bevorzugt mit 5 bis 20, insbesondere mit 7 bis 10 theoretische Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators kann vorzugsweise von 12 bis 36, insbesondere von 20 bis 30 theoretische Trennstufen umfassen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Isobuten-Umsatz von 30 bis 98 %, vorzugsweise von 50 bis 95 % und besonders bevorzugt von 75 bis 90 % bezogen auf den Isobutengehalt im Zulauf zur Reaktivdestillation erreicht wird.

Die hydraulische Belastung in der katalytischen Packung der Kolonne beträgt vorzugsweise 10 % bis 110 %, bevorzugt 20 % bis 90 % und besonders bevorzugt 35 bis 75 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne - z. B. der Böden (Vauck/Müller, "Grundoperationen chemischer Verfahrenstechnik", S. 626, VEB Deutscher Verlag für Grundstoffindustrie.). Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen kleiner als 1,5 betrieben, insbesondere mit solchen, die größer 0,6 und kleiner 1,2, bevorzugt größer 0,7 und kleiner 1,1 betragen.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht. Es ist im erfindungsgemäßen Verfahren aber auch möglich, diese Bereiche räumlich zu trennen, ohne auf die Vorteile einer Reaktivdestillation zu verzichten.

In einer Verfahrensvariante kann die Reaktivdestillationskolonne als Destillationskolonne mit einem oder mehreren außenliegenden Reaktoren, die den Katalysator enthalten und im Nebenstrom betrieben werden, sogenannten Seitenreaktoren, ausgeführt sein.

Das Kopfprodukt der Reaktivdestillationskolonne enthält im Wesentlichen ein C₄-Kohlenwasserstoffgemisch und Ethanol.

Als Katalysator wird vorzugsweise sowohl in den Festbettstufen als auch in einer gegebenenfalls vorhandenen Reaktivdestillationskolonne, ein fester Stoff, der weder im Einsatzstoffgemisch noch im Produktgemisch löslich ist, mit sauren Zentren an seiner Oberfläche eingesetzt. Der Katalysator sollte unter Reaktionsbedingungen keine sauren Stoffe an das Produktgemisch abgeben, weil dies zu Ausbeuteverluste führen kann.

Die Aktivität der Katalysatoren wird vorzugsweise so gewählt, dass sie unter Reaktionsbedingungen die Addition von Ethanol an Isobuten katalysieren, jedoch kaum die Addition an lineare Butene. Weiterhin sollten die Katalysatoren die Oligomerisierung von linearen Butenen und die Dialkyletherbildung aus zwei Molekülen eingesetzten Ethanol möglichst nicht oder nur wenig katalysieren. Im Hinblick auf eine hohe Ausbeute an 1-Buten sollte die Aktivität für die Isomerisierung von 1-Buten zu 2-Buten vorzugsweise gering sein.

Als feste Katalysatoren können beispielsweise Zeolithe, säureaktivierte Bentonite und/oder Tonerden, sulfonierte Zirkoniumoxide, Montmorillonite oder saure Ionenaustauscherharze verwendet werden.

Eine im erfindungsgemäßen Verfahren in Verfahrensschritt a) bevorzugte Gruppe von eingesetzten sauren Katalysatoren sind feste Ionenaustauscherharze, insbesondere solche mit Sulfonsäuregruppen. Geeignete Ionenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind: Styrol, Vinyltoluol, Vinylnaphthalin, Vinylethylbenzol, Methylstyrol, Vinylchlorbenzol, Vinylxylol und Divinylbenzol. Insbesondere werden die Cooligomeren, die durch Umsetzung von Styrol mit Divinylbenzol entstehen, als Vorstufe für die Herstellung von Ionenaustauscherharzen mit Sulfonsäuregruppen verwendet. Die Harze können gelförmig, makroporös oder schwammförmig hergestellt werden.

Die Eigenschaften dieser Harze, insbesondere spezifische Oberfläche, Porosität, Stabilität, Quellung bzw. Schrumpfung und Austauschkapazität, können durch den Herstellprozess variiert werden.

Im erfindungsgemäßen Verfahren können die Ionenaustauscherharze in ihrer H-Form eingesetzt werden. Stark saure Harze des Styrol-Divinylbenzol-Typs werden u. a. unter folgenden Handelsnamen verkauft: Duolite C20, Duolite C26, Amberlyst 15, Amberlyst 35, Amberlite IR-120, Amberlite 200, Dowex 50, Lewatit SPC 118, Lewatit SPC 108, K2611, K2621, OC 1501.

Das Porenvolumen beträgt vorzugsweise von 0,3 bis 0,9 ml/g, insbesondere von 0,5 bis 0,9 ml/g. Die Korngröße des Harzes beträgt bevorzugt von 0,3 mm bis 1,5 mm, insbesondere von 0,5 mm bis 1,0 mm. Die Korngrößenverteilung kann enger oder weiter gewählt werden. So können beispielsweise Ionenaustauscherharze mit sehr einheitlicher Korngröße (monodisperse Harze) eingesetzt werden. Die Kapazität des Ionenaustauscher beträgt, bezogen auf die Lieferform, vorzugsweise von 0,7 bis 2,0 eq/l, insbesondere von 1,1 bis 2,0 eq/l, bzw. vorzugsweise von 0,5 bis 5,5 mol/kg, insbesondere von 0,8 bis 5,5 mol/kg (Die Angaben zur Kapazität in mol/kg beziehen sich auf das jeweils bis zur Gewichtskonstanz im warmen Stickstoffstrom bei z. B. 105 °C getrocknete Ionentauscherharz.).

Im Reaktionsteil einer gegebenenfalls in Verfahrensschritt a) vorhandenen Reaktivdestillation können die gleichen Katalysatoren eingesetzt werden, wie sie in den einfachen Reaktoren eingesetzt werden. In der Reaktivdestillationskolonne kann der Katalysator entweder in der Packung integriert sein, beispielsweise KataMax® (wie in EP 0 428 265 beschrieben), KataPak® (wie in EP 0 396 650 oder DE 298 07 007.3 U1 beschrieben) oder auf Formkörpern aufpolymerisiert sein (wie in US 5 244 929 beschrieben).

### Verfahrensschritt b)

Die Abtrennung der nicht umgesetzten C₄-Kohlenwasserstoffe III aus dem Austrag des Schrittes a) in Schritt b) erfolgt durch thermische Trennverfahren, beispielweise durch Destillationen oder Fraktionierungen. Beinhaltet der Verfahrensschritt a) eine Reaktivdestillation, so kann der Verfahrensschritt b) teilweise oder vollständig bereits bei der Durchführung der Reaktivdestillation stattfinden und ein separater Schritt b) kann gegebenenfalls entfallen.

Der Verfahrensschritt b) kann vorzugsweise in einer Destillationskolonne durchgeführt werden. Vorzugsweise weist die Destillationskolonne eine Anzahl an theoretischen Trennstufen von 25 bis 50, bevorzugt 30 bis 40 auf. Der Zulauf zu dieser Kolonne erfolgt vorzugsweise im Bereich der 10-ten bis 15-ten theoretischen Trennstufe (von oben). Die Destillation im Verfahrensschritt a) wird vorzugsweise bei einem Druck von 3 bis 10, bevorzugt von 4 bis 7 bara, einer bevorzugten Kopftemperatur von 30 bis 70 °C, besonders bevorzugt von 45 bis 60 °C und einer bevorzugten Sumpftemperatur von 105 bis 125 °C, besonders bevorzugt von 110 bis 120 °C durchgeführt.

Das thermische Trennverfahren wird vorzugsweise so durchgeführt, dass als Sumpfprodukt eine im wesentlichen ETBE aufweisende Fraktion und als Kopfprodukt eine im Wesentlichen nicht umgesetzte C₄-Kohlenwasserstoffe sowie Ethanol enthaltende Fraktion erhalten wird. Werden die Butenoligomeren vor der Umsetzung mit Ethanol abgetrennt, besteht das Sumpfprodukt der Reaktivdestillationskolonne bevorzugt aus ETBE.

Das Kopfprodukt des Verfahrensschrittes b), also das Kopfprodukt der Reaktivdestillation oder der thermischen Trennung, kann direkt einer weiteren Auftrennung gemäß Verfahrensschritt c) zugeführt werden oder aber zunächst in einem oder mehreren Aufarbeitungsschritten aufgearbeitet werden.

### Verfahrensschritt f)

Es kann vorteilhaft sein, wenn das erfindungsgemäße Verfahren zwischen den Verfahrensschritten b) und c) einen weiteren Verfahrensschritt f) aufweist, in dem aus dem Kopfprodukt des Verfahrensschrittes b) also dem Kopfprodukt aus der Reaktivdestillation oder aus der thermischen Trennung, welches im Wesentlichen nicht umgesetzte C₄-Kohlenwasserstoffe und Ethanol aufweist, zunächst das Ethanol vollständig oder nahezu vollständig von den nicht umgesetzten C₄-Kohlenwasserstoffen abgetrennt wird.

Bevorzugt wird das Kopfprodukt aus dem Verfahrensschritt b) welches am Kopf der Destillationskolonne oder Reaktivdestillationskolonne erhalten wird, in eine Extraktionskolonne überführt, in die im Gegenstrom ein Extraktionsmittel, wie z. B. Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion der Strom aus in Stufe a) und gegebenenfalls b) nicht umgesetzten Kohlenwasserstoffen III erhalten. Dieses Produkt kann in Verfahrensschritt c) eingespeist werden.

Der Verfahrensschritt f) kann vorzugsweise in einer Extraktionskolonne durchgeführt werden. Vorzugsweise weist die Extraktionskolonne von 5 bis 20, bevorzugt von 10 bis 15 theoretische Trennstufen auf. Die Extraktion im Verfahrensschritt f) wird vorzugsweise bei einem Druck von 5 bis 12, bevorzugt von 7 bis 10 bara. Die Extraktion im Verfahrensschritt f) wird vorzugsweise bei einer Temperatur von 30 bis 60 °C und bevorzugt von 35 bis 45 °C durchgeführt. Dass Verhältnis von Extraktionsmittel, vorzugsweise Wasser zum Kopfprodukt aus dem Verfahrensschritt b) bzw. a) beträgt vorzugsweise von 0,05 bis 0,5, bevorzugt von 0,1 bis 0,25 und besonders bevorzugt von 0,15 bis 0,2.

Das im Sumpf der Extraktionskolonne anfallende mit Ethanol angereicherte Extraktionsmittel kann destillativ aufgetrennt werden und das so erhaltene Ethanol, wenn als Extraktionsmittel Wasser eingesetzt wurde, gegebenenfalls nach einer Trocknung, dem Prozess als Ausgangsstoff in den Schritte a) oder d) wieder zugeführt werden.

### Verfahrensschritt c)

Nach der Abtrennung des ETBE und gegebenenfalls nach Abtrennung des nicht umgesetzten Ethanols, wird der aus Schritt b) oder f) erhaltene Kohlenwasserstoffstrom in Schritt c) destillativ getrennt. Die destillative Trennung wird so durchgeführt, dass eine mindestens 1-Buten und Isobuten enthaltende Fraktion IV (Kopffraktion) und eine nahezu Isobuten-freie, vorzugsweise weniger als 5 Massen-%, bevorzugt weniger als 1 Massen-% und besonders bevorzugt weniger als 0,1 Massen-% Isobuten aufweisende, mindestens 2-Butene und n-Butan enthaltende Fraktion V (Sumpffraktion), erhalten wird. Die Fraktion V enthält mindestens 95 Massen-%, vorzugsweise mindestens 99 Massen-%, besonders bevorzugt mindestens 99,8 Massen-% der ursprünglich im als Produkt des Schrittes c) erhaltenen Kohlenwasserstoffstroms enthaltenen 2-Butene. Bevorzugt weist die Fraktion IV weniger als 1 Massen-%, besonders bevorzugt weniger als 0,2 Massen-% an n-Butan auf. Die destillative Trennung kann in für die Auftrennung solcher Kohlenwasserstoffgemische üblicherweise eingesetzten Apparaturen durchgeführt werden. Solche Apparaturen können z. B. Destillations- oder Fraktionierungskolonnen sein.

Die Fraktion V kann als Alkylierungsmittel eingesetzt werden. Insbesondere kann sie als Ausgangsstoff für die Herstellung von n-Buten-Oligomeren, wie z. B. Di-n-buten oder Tributen, eingesetzt werden. Ein Oligomerisierungsverfahren, bei dem die Fraktion V eingesetzt werden kann, ist z. B. das OCTOL-Verfahren der OXENO Olefinchemie GmbH, wie es in DE 196 29 906 oder EP 0 395 857 beschrieben wird.

Bevorzugt wird die Trennung in einer Superfraktionierkolonne durchgeführt. Der Zulauf zu dieser Kolonne erfolgt vorzugsweise in der unteren Hälfte, bevorzugt im unteren Drittel der Kolonne. Wegen der engen Siedelage des zu trennenden Gemisches wird die Destillation vorzugsweise in einer Kolonne mit vorzugsweise mehr als 100, bevorzugt mehr als 125, besonders bevorzugt mit 150 oder mehr theoretischen Trennstufen und ganz besonders bevorzugt mit 150 bis 200 theoretischen Trennstufen durchgeführt. Das Rücklaufverhältnis (Rücklaufmenge zu Destillatabnahme) beträgt, in Abhängigkeit von der realisierten Stufenzahl und vom Betriebsdruck, vorzugsweise kleiner-gleich 20, bevorzugt kleiner-gleich 14, besonders bevorzugt kleiner-gleich 11 und ganz besondere bevorzugt von 8 bis 11. Die Kondensation kann gegen Kühlwasser oder Luft durchgeführt werden. Der Destillatbehälter wird vorzugsweise als flüssig-flüssig-Abscheider ausgeführt. Dadurch kann gegebenenfalls im Zulaufstrom enthaltenes Wasser als zweite Phase im Destillatbehälter abgetrennt werden und es kann ein technisch wasserfreies Sumpfprodukt erhalten werden.

Die Trennung gemäß Verfahrensschritt c) wird vorzugsweise bei einem Druck von 4 bis 10 bara, bevorzugt bei einem Druck von 5 bis 7 bara durchgeführt. Die Temperatur bei welcher die Trennung durchgeführt wird beträgt vorzugsweise von 35 bis 65 °C, bevorzugt 40 bis 50 °C.

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren, aber auch eine Kolonnen, die außerhalb des erfindungsgemäßen Verfahren am Anlagenstandort vorhanden ist, mit der erfindungsgemäßen Kolonne des Verfahrensschrittes c) verschaltet werden.

### Verfahrensschritt d)

Die aus Schritt c) erhaltene Isobuten-haltige Fraktion IV wird im erfindungsgemäßen Verfahren in einem weiteren Reaktionsschritt (Schritt d) umgesetzt, bei der das restliche Isobuten durch Anlagerung von Ethanol zum ETBE umgesetzt wird.

Die Veretherung des Isobutens wird ebenso wie die Veretherung gemäß Schritt a) als sauer katalysierte Reaktion durchgeführt. Als Ethanol kann hochreiner Ethanol, reiner Ethanol oder Ethanol verwendet werden, der geringe Mengen an Verunreinigungen aufweist. Bevorzugt liegt die Reinheit des eingesetzten Ethanols, angegeben in Massen-% an Ethanol, größer 90 %, besonders bevorzugt größer 95 % und ganz besonders bevorzugt bei gleich oder größer 99 %. Ethanol mit einer Reinheit von größer-gleich 99 Massen-% wird in Europa beispielsweise als Bioethanol angeboten. Der Gehalt an Wasser liegt bevorzugt unter 3 Massen-%, besonders bevorzugt unter 1 Massen-%, ganz besonders bevorzugt unter 0,5 Massen-%. Es kann im erfindungsgemäßen Verfahren vorteilhaft sein, vergälltes Ethanol einzusetzen. Besonders bevorzugt wird als Ethanol ein Ethanol eingesetzt, welches als Vergällungsmittel ETBE, vorzugsweise in einer Konzentration von 0 bis 5 Massen-%, bevorzugt von 0,005 bis 1 Massen-%, besonders bevorzugt 0,05 bis 1 Massen-% und ganz besonders bevorzugt von 0,01 bis 0,2 Massen-% aufweist. In Deutschland wird vorzugsweise Ethanol eingesetzt, welches 0,1 bis 1 Massen-% an Vergällungsmittel aufweist. Durch die Verwendung von mit ETBE vergälltem Ethanol wird vermieden, dass Fremdstoffe in den Prozess eingetragen werden.

Für die Umsetzung von Isobuten mit Alkoholen, insbesondere mit Methanol zu Methyl-tert.-butylether wurden diverse Verfahrensvarianten entwickelt (vergleiche: Ullmann's Encyclopedia of Industrial Chemistry, Online Version, 2004, Wiley & Sons, Stichwort Methyl-tert-Butylether, und dort zitierte Literatur; Obenaus, Fritz; Droste, Wilhelm, Erdoel & Kohle, Erdgas, Petrochemie (1980), 33(6), 271 bis 275; DE 26 29 769; DE 28 53 769). Prinzipiell sind alle bekannten Verfahren zur Umsetzung des Isobutens mit Alkoholen geeignet als Verfahrensschritt d) im Rahmen der vorliegenden Erfindung eingesetzt zu werden.

Bevorzugt werden Verfahren, bei denen die Umsetzung in flüssiger Phase an einem sauren Ionenaustauscherharz erfolgt, eingesetzt. Als Reaktoren, in denen der Ethanol mit dem Isobuten bis nahe an das thermodynamische Gleichgewicht umgesetzt wird, können herkömmliche Festbettreaktoren (Rohrbündelreaktoren, adiabatische Festbettreaktoren, Kreislaufreaktoren) eingesetzt werden. Sie können mit oder ohne partieller Rückführung, wobei gegebenenfalls der Rückführungsstrom gekühlt werden kann, betrieben werden.

Besonders bevorzugt wird in Schritt d) ein Reaktorsystem eingesetzt, das zwei Reaktoren, insbesondere Festbettreaktoren aufweist. Vorzugsweise werden beide Reaktoren im geraden Durchgang betrieben.

Die Reaktoren können bei Temperaturen von 25 bis 110 °C, bevorzugt bei Temperaturen von 30 bis 70 °C und besonders bevorzugt bei Temperaturen von 35 bis 50 °C betrieben werden. Der Druck beträgt vorzugsweise 5 bis 50 bara, bevorzugt 10 bis 20 bara und besonders bevorzugt 10 bis 13 bara. Da das thermodynamische Gleichgewicht zwischen Ethanol/Isobuten und Ether bei tiefer Temperatur überwiegend auf der Seite des Ethers liegt, ist es bei Einsatz mehrerer Reaktoren bevorzugt, den ersten der Reaktoren bei höherer Temperatur (hohe Reaktionsgeschwindigkeit) als die Folgenden (Ausnutzung der Gleichgewichtslage) zu betreiben.

Das molare Verhältnis von Ethanol zu Isobuten im Zulauf zum Verfahrensschritt d) liegt vorzugsweise im Bereich von 25 zu 1 bis 1 zu 1, besonders bevorzugt von 15 zu 1 bis 3 zu 1 und besonders bevorzugt im Bereich von 10 zu 1 bis 5 zu 1.

Als Katalysatoren können bevorzugt solche eingesetzt werden, wie sie für den Verfahrensschritt a) beschrieben werden, wobei das erfindungsgemäße Verfahren so durchgeführt werden kann, dass in Schritt a) und d) jeweils der gleiche Katalysator oder unterschiedliche Katalysatoren eingesetzt werden können. Vorzugsweise werden in den Schritten a) und d) die gleichen Katalysatoren eingesetzt.

In einer bevorzugten Ausführungsform wird die Addition des Ethanols an das Isobuten in Gegenwart eines sauren Katalysators so durchgeführt, dass zumindest eine Reaktionsstufe als Reaktivdestillation durchgeführt wird. Besonders bevorzugt wird die sauer katalysierte Veretherung in Schritt d) in mindestens zwei Reaktionsstufen durchgeführt, wobei vorzugsweise zumindest eine, besonders bevorzugt die letzte Reaktionsstufe als Reaktivdestillation durchgeführt wird. In dem/den Festbettreaktor/en wird dabei zunächst an einem sauren Katalysator aus der Isobuten-haltigen Fraktion IV und dem Ethanol VI ein Reaktionsgemisch hergestellt, das hinsichtlich seiner Isobuten-, Ethanol- und tert.-Butylether-Konzentration in der Nähe des thermodynamischen Gleichgewichts liegt. In diesem Reaktionsschritt wird der Restgehalt an Isobuten vorzugsweise so weit umgesetzt, dass mit der nachfolgenden Reaktivdestillation die erforderliche Reinheit des 1-Butens erreicht werden kann. Dieses Gemisch wird in der nächsten/letzten Reaktionsstufe in die Reaktivdestillationskolonne eingespeist, wo ein weiterer Teil des Isobutens zum Ether umgesetzt wird. Ganz besonders bevorzugt wird der Schritt d) in einem Reaktorsystem durchgeführt, das zwei in Reihe geschaltete Reaktoren und eine Reaktivdestillationskolonne aufweist, wobei die beiden Reaktoren vorzugsweise im geraden Durchgang betrieben werden, und der Austrag aus dem zweiten Reaktor in die Reaktivdestillationskolonne eingespeist wird.

Im Reaktionsteil der Reaktivdestillationskolonne können die gleichen Katalysatoren, wie die oben für die einfache Ausführungsform der Verfahrensstufe ohne die Verwendung einer Reaktivdestillation beschriebenen, eingesetzt werden.

In der Reaktivdestillationskolonne kann der Katalysator entweder in der Packung integriert sein, beispielsweise KataMax^{®} (wie in EP 0 428 265 beschrieben), KataPak^{®} (wie in EP 0 396 650 oder DE 298 07 007.3 U1 beschrieben) oder auf Formkörpern aufpolymerisiert (wie in US 5 244 929 beschrieben) sein.

Die Umsetzung des Isobutens mit Ethanol zu ETBE erfolgt in der Reaktivdestillation im Temperaturbereich von 10 bis 140 °C, bevorzugt bei 30 bis 70 °C, besonders bevorzugt bei 35 bis 50 °C (Temperatur im Bereich der Kolonne, in der sich der Katalysator befindet. Die Sumpftemperatur der Kolonne kann deutlich höher liegen).

Insbesondere wird das ETBE durch Umsetzung mit Ethanol in einer Weise hergestellt, wie es in DE 101 02 082 für die Reaktion von Methanol mit Isobuten zu MTBE beschrieben wird. Das Isobuten aufweisende C₄-Kohlenwasserstoffgemisch wird zusammen mit Ethanol in den/die Vorreaktor(en) eingespeist. Dabei wird der Ethanol bevorzugt im Überschuss eingesetzt. In den Vorreaktoren entsteht ein Gemisch, in dem Isobuten, Ethanol und ETBE im Gleichgewicht oder nahezu im Gleichgewicht stehen. Dieses Reaktionsgemisch wird in die Reaktivdestillationskolonne eingeleitet.

Im Zulauf der Reaktivdestillationskolonne kann mehr Ethanol enthalten sein, als für die vollständige Umsetzung des noch vorhandenen Isobutens gebraucht wird. Der Alkoholüberschuss sollte jedoch derart bemessen werden, dass eine ausreichende Ethanolmenge für das sich bildende Azeotrop aus Ethanol und C₄-Kohlenwasserstoffen vorhanden ist.

Optional kann, beispielsweise wenn der Ethanolgehalt im Kolonnenzulauf unter dem maximal zulässigen Wert liegt, zusätzlicher Ethanol zum Kolonnenzulauf zugemischt werden. Darüber hinaus kann am Kopf der Reaktivdestillationskolonne oberhalb des Kolonnenzulaufs unterhalb eines Flüssigkeitsverteilers oder in einem Flüssigkeitsverteiler oberhalb oder im Bereich der Reaktivzone, vorzugsweise im Bereich der Reaktivzone über eine gesonderte Einrichtung eine Ethanol-Einspeisung erfolgen. Eine zusätzliche Einspeisung von Ethanol kann z. B. in den Rücklauf der Kolonne oder direkt in die reaktiven Packungen erfolgen. Die zusätzliche Ethanolzugabe sollte so bemessen sein, dass in den Packungen der Reaktivzone der Ethanolgehalt in der Flüssigphase vorzugsweise größer-gleich 1,5 Massen-%, bevorzugt größer-gleich 2 Massen-% und besonders bevorzugt von 2 bis 3 Massen-% beträgt.

Bevorzugt weist die Reaktivdestillationskolonne oberhalb der Katalysatorpackung einen Bereich rein destillativer Trennung auf, besonders bevorzugt mit 5 bis 20, insbesondere mit 7 bis 10 theoretische Trennstufen. Die Katalysatorzone lässt sich mit einer destillativen Wirkung von 1 bis 5 theoretischen Trennstufen pro Meter Packungshöhe abschätzen. Die Trennzone unterhalb des Katalysators kann vorzugsweise von 12 bis 36, insbesondere von 20 bis 30 theoretische Trennstufen umfassen. Die Höhe der Katalysatorzone/Reaktivzone lässt sich in Abhängigkeit vom gewünschten Isobuten-Umsatz durch einfache Vorversuche ermitteln. Die Katalysatormenge wird vorzugsweise so groß gewählt, dass ein Restisobutengehalt im Kopfprodukt von kleiner 2000 Massen-ppm (wppm), bevorzugt kleiner 1500 wppm erreicht wird.

Der Zulauf zur Reaktivdestillationskolonne kann oberhalb oder unterhalb der Katalysatorzone erfolgen. Der Zulauf zur Reaktivdestillationskolonne erfolgt vorzugsweise unterhalb der reaktiven Packung, bevorzugt 3 bis 13, besonders bevorzugt 4 bis 10 theoretische Trennstufen unterhalb der reaktiven Packung.

Die Reaktivdestillationskolonne wird bei Drücken, gemessen am Kolonnenkopf, von 3 bara bis 10 bara, bevorzugt 4 bara bis 7 bara betrieben, insbesondere von 5 bara bis 6 bara. Die hydraulische Belastung in der katalytischen Packung der Kolonne beträgt vorzugsweise 10 % bis 110 %, bevorzugt 20 % bis 90 % und besonders bevorzugt 35 bis 75 % ihrer Flutpunktbelastung. Unter hydraulischer Belastung einer Destillationskolonne wird die gleichmäßige strömungstechnische Beanspruchung des Kolonnenquerschnitts durch den aufsteigenden Dampf-Massenstrom und den rücklaufenden Flüssigkeits-Massenstrom verstanden. Die obere Belastungsgrenze kennzeichnet die maximale Belastung durch Dampf und Rücklaufflüssigkeit, oberhalb derer die Trennwirkung infolge Mitreißens oder Stauens der Rücklaufflüssigkeit durch den aufsteigenden Dampfstrom absinkt. Die untere Belastungsgrenze kennzeichnet die minimale Belastung, unterhalb derer die Trennwirkung absinkt oder zusammenbricht infolge unregelmäßiger Strömung oder Leerlaufen der Kolonne ***-*** z. B. der Böden (Vauck/Müller, "Grundoperationen chemischer Verfahrenstechnik", S. 626, VEB Deutscher Verlag für Grundstoffindustrie.).

Am Flutpunkt werden die vom Gas an die Flüssigkeit übertragenen Schubspannungen so groß, dass die gesamte Flüssigkeitsmenge in Form von Tropfen mit dem Gas mitgerissen wird oder dass es zu Phaseninversion in der Kolonne kommt (J. Mackowiak, "Fluiddynamik von Kolonnen mit modernen Füllkörpern und Packungen für Gas/Flüssigkeitssysteme", Otto Salle Verlag 1991).

Die Reaktivdestillationskolonne wird vorzugsweise mit Rücklaufverhältnissen von 0,2 bis 4 betrieben, insbesondere mit solchen, die von 0,4 bis 2, bevorzugt von 0,5 bis 1 betragen.

Wird in der Stufe d) als letzter Schritt eine Reaktivdestillationskolonne eingesetzt, so kann in dieser zumindest teilweise der Schritt d) sowie der Schritt e), nämlich die Abtrennung des ETBE von den nicht umgesetzten Kohlenwasserstoffen stattfinden. Auf einen weiteren Schritt e) kann dann gegebenenfalls verzichtet werden.

Unter den Oberbegriff der Reaktivdestillation fallen alle verfahrenstechnischen Maßnahmen, bei denen Destillation und Reaktion gleichzeitig durchgeführt werden. In den beschriebenen Reaktoren wird dies durch eine besondere Ausführung der Packungen in einer Kolonne erreicht. Es ist im erfindungsgemäßen Verfahren aber auch möglich, diese Bereiche räumlich zu trennen, ohne auf die Vorteile einer Reaktivdestillation zu verzichten.

In einer Verfahrensvariante wird die Reaktivdestillationskolonne als Destillationskolonne mit einem oder mehreren außenliegenden Reaktor(en), der/die den Katalysator enthält/enthalten und im Nebenstrom betrieben wird/werden, ausgeführt.

### Verfahrensschritt e)

Wird in Verfahrensschritt d) keine Reaktivdestillationskolonne zur Veretherung und gleichzeitigen Trennung eingesetzt, so muss im erfindungsgemäßen Verfahren ein eigenständiger Schritt e) vorgesehen werden, in welchem das Produkt aus dem Verfahrensschritt d) getrennt wird in den ETBE enthaltenden Sumpfstrom und einen Strom, der die nicht umgesetzten Kohlenwasserstoffe aufweist. Ansonsten erfolgt die destillative Trennung gemäß Verfahrensschritt e) in der Reaktivdestillationskolonne. Die Trennung kann z. B. dadurch erfolgen, dass der Austrag aus dem Reaktor des Verfahrensschrittes d) in eine Destillationskolonne eingespeist wird. Die Kolonne kann mit einem Sumpfverdampfer und einem Kondensator für das Kopfprodukt ausgerüstet sein. Als Sumpfprodukt der Destillationskolonne wird ETBE und gegebenenfalls überschüssiger Ethanol erhalten. Das Kopfprodukt kann teilweise als Rücklauf in die Kolonne zurückgefahren werden. Der andere Teil, kann dem Verfahrensschritt h) zugeführt werden.

Die Kolonne weist vorzugsweise mehr als 20, bevorzugt mehr als 25, besonders bevorzugt von 30 bis 50 theoretischen Trennstufen auf. Das Rücklaufverhältnis ist, in Abhängigkeit von der realisierten Stufenzahl, vorzugsweise kleiner-gleich 1. Besonders bevorzugt wird das Rücklaufverhältnis auf einen Wert von 0,9 bis 0,6 eingestellt. Die Kondensation kann gegen Kühlwasser oder Luft durchgeführt werden. Zur Beheizung des Verdampfers der Kolonne kann z. B. Dampf eingesetzt werden. Es kann vorteilhaft sein, den Zulaufstrom zur Kolonne zumindest teilweise vorverdampft in die Kolonne zu leiten oder direkt in die Kolonne zu flashen. Bevorzugt wird dafür dem Zulaufstrom in einem externen Wärmeübertrager Wärme, z. B. durch Nutzung von Abwärme, zugeführt. Zum Erzielen einer teilweisen Verdampfung ist ein Kettle-Verdampfer die bevorzugte Ausführungsform des Wärmeübertragers. Es kann außerdem vorteilhaft sein, wenn ein mit Prozess- oder Abwärme auf niedrigerem Temperaturniveau beheizter Zwischenverdampfer im unteren Teil der Kolonne eingesetzt wird.

Der Zulauf zu der Kolonne erfolgt in Verfahrensschritt e) vorzugsweise auf der 10 bis 15 theoretischen Trennstufe. Die Kolonne wird vorzugsweise mit einem Druck von 4 bis 11, bevorzugt von 5 bis 8 bara betrieben. Die Kopftemperatur der in Verfahrensschritt e) eingesetzten Kolonne beträgt vorzugsweise von 40 bis 70 °C, bevorzugt von 45 bis 60 °C.

Weist das Kopfprodukt von Verfahrensschritt e) unabhängig davon, ob der Schritt in einer Destillations- oder Reaktivdestillationskolonne durchgeführt wurde, noch Restmengen an Ethanol in den C₄-Kohlenwasserstoffen auf, so kann es vorteilhaft sein, diese in mindestens einem zusätzlichen Extraktionsschritt mit Wasser auszuwaschen. Diese Wäsche kann nach den bekannten Standardverfahren der Technik, beispielsweise in einer Extraktionskolonne oder in einer Kaskade von Mixern und Trennbehältern, durchgeführt werden (Siehe Verfahrensschritt h)).

Das Sumpfprodukt, welches ETBE und gegebenenfalls überschüssiges Ethanol enthält, kann direkt verwendet werden oder einer Aufarbeitung, z. B. einer weiteren Destillation zugeführt werden, in welcher das ETBE von den übrigen Bestandteilen abgetrennt wird.

In einer weiteren bevorzugten Ausführungsform des Verfahrens wird das in der Reaktivdestillation bzw. Destillation der Schritte d) bzw. e) anfallende Sumpfprodukt, welches ETBE und gegebenenfalls nicht umgesetzten Ethanol enthält, ganz oder teilweise in den Schritt a) und/oder b) zurückgeführt. Diese Ausführungsform ist insbesondere dann vorteilhaft, wenn die Umsetzung in Schritt d) mit einem stöchiometrischen Überschuss an Ethanol betrieben wird. Bevorzugt wird bei dieser Variante in Schritt d) die Umsetzung nur in Festbettreaktoren durchgeführt und in Schritt e) eine destillative Trennung in einer Destillationskolonne durchgeführt. Die nicht umgesetzten C₄-Kohlenwasserstoffe VIII werden als Kopfprodukt erhalten und das anfallende Sumpfprodukt, welches mindestens ETBE und gegebenenfalls nicht umgesetzten Ethanol enthält, wird ganz oder teilweise in den Schritt a) und/oder b) zurückgeführt. Wird der Verfahrensschritt d) mit einem großen Ethanolüberschuss durchgeführt, z. B. mit einem molaren Verhältnis von Ethanol zu Isobuten von größer 2 zu 1, insbesondere größer 5 zu 1, so fällt als Sumpfprodukt in Schritt e) eine Mischung an, die im wesentlichen Ethanol enthält und deshalb besonders gut als Einsatzstoff in die Stufe a) zurückgeführt werden kann. Bei dieser Ausführungsform wird das ETBE ausschließlich als Sumpfprodukt des Verfahrenschrittes b) erhalten. Zur destillativen Aufarbeitung des Sumpfproduktes aus Verfahrenschritt e) wird bei dieser Ausführungsform der Verfahrensschritt b) genutzt.

### Verfahrensschritt h)

Bevorzugt wird das Kopfprodukt aus dem Verfahrensschritt d) bzw. e), welches am Kopf der Destillationskolonne oder Reaktivdestillationskolonne erhalten wird, in eine Extraktionskolonne überführt, in die im Gegenstrom ein Extraktionsmittel, wie z. B. Wasser, über einen am Kopf befindlichen Zulauf eingespeist wird. Das Extraktionsmittel kann über den Ablauf am Sumpf der Kolonne entnommen werden. Am Kopf der Kolonne wird als Produkt der Extraktion der Strom aus in Stufe d) und gegebenenfalls e) nicht umgesetzten Kohlenwasserstoffen VIII erhalten. Dieser kann einer weiteren Verwendung beispielsweise einer Aufarbeitung zu 1-Buten (Verfahrensschritt i)) zugeführt werden. Das im Sumpf der Kolonne anfallende mit Ethanol angereicherte Extraktionsmittel kann destillativ aufgetrennt werden und das Ethanol gegebenenfalls, wenn als Extraktionsmittel Wasser eingesetzt wurde, nach einer Trocknung dem Prozess als Ausgangsstoff in die Schritte a) oder d) zurückgeführt werden. Durch diese Maßnahme kann eine gegebenenfalls vorhandene nachfolgende Kolonne (Verfahrensschritt i)) bei niedrigerem Druck betrieben werden. Ohne Extraktion müsste ein nachfolgende Kolonne bei höherem Druck betrieben werden, um Ethanol über Kopf abtrennen zu können.

Der Verfahrensschritt h) kann vorzugsweise in einer Extraktionskolonne durchgeführt werden. Vorzugsweise weist die Extraktionskolonne von 5 bis 20, bevorzugt 10 bis 15 theoretische Trennstufen auf. Die Extraktion im Verfahrensschritt h) wird vorzugsweise bei einem Druck von 5 bis 12, bevorzugt von 7 bis 10 bara durchgeführt. Die Extraktion im Verfahrensschritt h) wird vorzugsweise bei einer Temperatur von 30 bis 60 °C, besonders bevorzugt von 35 bis 45 °C durchgeführt. Dass Verhältnis von Extraktionsmittel, insbesondere Wasser zum Kopfprodukt aus dem Verfahrensschritt d) bzw. e) beträgt vorzugsweise von 0,05 bis 0,5, bevorzugt von 0,1 bis 0,25 und besonders bevorzugt von 0,15 bis 0,2.

### Verfahrensschritt i)

Aus dem aus der Reaktivdestillation bzw. Destillation gemäß Schritt e) erhaltenen und gegebenenfalls von Ethanol befreiten C₄-Kohlenwasserstoffgemisch VIII aus nicht umgesetzten Kohlenwasserstoffen, das im wesentlichen 1-Buten, Isobutan und Leichtsieder aufweist, kann destillativ 1-Buten als weiteres Wertprodukt abgetrennt werden. Die Abtrennung des 1-Butens erfolgt vorzugsweise durch Destillation des Gemisches VIII in einer oder mehreren Destillationskolonnen.

In einer bevorzugten Ausführungsform erfolgt die Abtrennung des 1-Butens in einer Destillationskolonne, in der als Sumpfprodukt sehr reines 1-Buten erhalten wird. Als Kopfprodukt wird eine Isobutan-reiche Fraktion erhalten, die zudem Leichtsieder (beispielsweise C₃-Kohlenwasserstoffe) enthalten kann.

Bevorzugt wird die Trennung in einer Superfraktionierkolonne durchgeführt. Der Zulauf zu dieser Kolonne erfolgt vorzugsweise in die obere Hälfte, bevorzugt in die untere Hälfte der oberen Hälfte der Kolonne. Wegen der engen Siedelage des zu trennenden Gemisches wird die Kolonne mit vorzugsweise mehr als 100, bevorzugt mehr als 125, besonders bevorzugt mit 150 oder mehr und ganz besonders bevorzugt mit 150 bis 200 theoretischen Trennstufen ausgeführt. Das Rücklaufverhältnis (Rücklaufmenge zu Destillatabnahme) beträgt, in Abhängigkeit von der realisierten Stufenzahl und vom Betriebsdruck, vorzugsweise kleiner-gleich 100, bevorzugt kleiner 70, besonders bevorzugt von 30 bis 60. Die Kondensation kann gegen Kühlwasser oder Luft durchgeführt werden. Der Destillatbehälter wird vorzugsweise als Flüssigkeit-Flüssigkeit-Abscheider ausgeführt. Dadurch kann gegebenenfalls im Zulaufstrom enthaltenes Wasser als zweite Phase im Destillatbehälter abgetrennt und es kann ein technisch wasserfreies Sumpfprodukt erhalten werden.

Die Trennung gemäß Verfahrensschritt i) wird vorzugsweise bei einem Druck von 5 bis 11 bara, bevorzugt bei einem Druck von 6 bis 8 bara durchgeführt. Die Kopftemperatur, bei welcher die Trennung durchgeführt wird, beträgt vorzugsweise von 35 bis 65 °C, bevorzugt 45 bis 50 °C. Wenn eine Wärmeintegration vorgesehen wird, kann es vorteilhaft sein, dass der Verfahrensschritt i) bei höherer Temperatur und damit höherem Druck durchgeführt wird.

Zur Beheizung des Verdampfers der Kolonne kann ein üblicher Wärmeträger, wie z. B. Dampf oder Warmwasser sowie bevorzugt Abwärme aus anderen Prozessen eingesetzt werden. In letzterem Fall kann es vorteilhaft sein, die Kolonne mit mehr als einem Verdampfer auszustatten. Die Kolonne wird bevorzugt als einfache Kolonne mit mindestens einem Verdampfer und mindestens einem Kondensator ausgerüstet. Wegen des hohen Energiebedarfs und der kleinen Temperaturdifferenz zwischen Sumpf und Kopf der Kolonne sind Energiesparschaltungen besonders bevorzugte Ausführungsformen. Exemplarisch sei hier auf die Methode der Brüdenverdichtung verwiesen. Eine weitere besonders bevorzugte Schaltung ist die Zweidruckschaltung (double effect distillation) in Integration mit einer zweiten Kolonne. Die zweite Kolonne kann bevorzugt eine parallelgeschaltete Kolonne mit gleicher oder unterschiedlicher Trennaufgabe sein. Dabei wird eine der Kolonnen bei so hohem Druck gefahren, dass ihre Kondensationstemperatur zur Beheizung der anderen Kolonne ausreicht. Bei der wärmetechnischen Verschaltung von Kolonnen mit unterschiedlichen Trennaufgaben kann prinzipiell jede geeignete Kolonne aus dem erfindungsgemäßen Verfahren, aber auch eine Kolonne, die außerhalb des erfindungsgemäßen Verfahren am Anlagenstandort vorhanden ist, mit der erfindungsgemäßen Kolonne des Verfahrensschrittes i) verschaltet werden. Besonders bevorzugt ist die zweite Kolonne die C₄-Trennkolonne aus Verfahrensschritt c). Dabei wird eine der Kolonnen bei so hohem Druck gefahren, dass ihre Kondensationstemperatur zur Beheizung der anderen Kolonne ausreicht.

In einer weiteren bevorzugten Ausführungsform werden in einer ersten Destillationskolonne Leichtsieder als Kopfprodukt abgetrennt, im Sumpf der Kolonne wird eine Mischung, die hauptsächlich 1-Buten und Isobutan enthält, erhalten. In einer zweiten Kolonne wird diese Sumpfmischung in 1-Buten, welches als Sumpfprodukt anfällt, und eine Isobutan-reiche Fraktion (Kopfprodukt), getrennt.

Mit dem erfindungsgemäßen Verfahren hergestelltes, reines 1-Buten enthält vorzugsweise weniger als 5000 wppm (Massen-ppm), bevorzugt weniger als 2000 wppm und besonders bevorzugt weniger als 1500 wppm Isobuten und ist ein gefragtes Zwischenprodukt. Es wird beispielsweise als Comonomer bei der Herstellung von Polyethylen (LLDPE oder HDPE) sowie von Ethylen-Propylen-Mischpolymeren eingesetzt. Es findet weiterhin Einsatz als Alkylierungsmittel und ist Ausgangsstoff für die Herstellung von Butan-2-ol, Butenoxid, Valeraldehyd. Eine weitere Verwendung des erfindungsgemäß hergestellten nahezu Isobutenfreien 1-Butens ist die Herstellung von n-Buten-Oligomeren, insbesondere nach dem Octol-Prozess.

In Verfahrensschritt i) fällt neben dem 1-Buten üblicherweise (je nach Ausgangszusammensetzung der C₄-Kohlenwasserstoffe) eine Isobutan-reiche Fraktion an. Diese kann weiter, vorzugsweise zu reinem Isobutan, aufgereinigt werden. Eine Aufreinigung zu reinem Isobutan kann beispielsweise durch vollständige Hydrierung der noch enthaltenen Alkene zu Alkanen und anschließende Destillation erfolgen. Das bei der Aufarbeitung gewonnene Isobutan hat vorzugsweise eine Reinheit von mindestens 90 Massen-% Isobutan, besonders bevorzugt 95 Massen-% Isobutan und enthält bevorzugt weniger als 1000 wppm, besonders bevorzugt weniger als 200 wppm Olefine.

### Einsatzstoffe

In dem erfindungsgemäßen Verfahren können alle üblicherweise zur Verfügung stehenden technischen C₄-Kohlenwasserstoffgemische eingesetzt werden. Geeignete Isobuten-haltige C₄-Ströme sind beispielsweise C₄-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus Skelettisomerisierung linearer Butene und Gemische, entstanden durch Metathese von Olefinen. Diese Techniken sind in der Fachliteratur beschrieben. (K.Weissermel, H.J. Arpe, Industrielle Organische Chemie, Wiley-VCH, 5. Auflage, 1998, Seite 23-24; 65-99; 122-124).

Bevorzugt eingesetzt werden C₄-Fraktionen aus Steamcrackern, die primär zur Produktion von Ethen und Propen betrieben werden und in denen als Rohstoffe beispielsweise Raffineriegase, Naphtha, Gasöl, LPG (liquified petroleum gas) und NGL (natural gas liquid) eingesetzt werden, oder Katcrackern. Die als Nebenprodukt anfallenden C₄-Schnitte enthalten je nach Crack-Verfahren unterschiedliche Mengen an Isobuten. Weitere Hauptbestandteile sind 1,3-Butadien, 1-Buten, c-2-Buten, t-2-Buten, n-Butan und i-Butan. Typische Isobuten-Gehalte in der C₄-Fraktion liegen bei C₄-Fraktionen aus Steam-Crackern bei 18 bis 35 Massen-%, bei -Fluid-Katcrackern (FCC) bei 10 bis 20 Massen-%.

Für das erfindungsgemäße Verfahren ist es vorteilhaft, mehrfach ungesättigte Kohlenwasserstoffe wie 1,3-Butadien aus dem Einsatzgemisch zu entfernen. Dies kann nach bekannten Verfahren, beispielsweise durch Extraktion, Extraktivdestillation oder Komplexbildung erfolgen (vgl. K.Weissermel, H.J. Arpe, Industrielle Organische Chemie, Wiley-VCH, 5. Auflage, 1998, Seiten 119 bis 121).

Eine Alternative zur Abtrennung der mehrfach ungesättigten Kohlenwasserstoffe ist eine selektive chemische Umsetzung. So kann beispielsweise 1,3-Butadien selektiv zu linearen Butenen hydriert werden, wie z. B. in EP 0 523 482 beschrieben. Auch durch selektive Umsetzungen des 1,3-Butadiens, zum Beispiel Dimerisierung zum Cyclooctadien, Trimerisierung zum Cyclododecatrien, Polymerisations- oder Telomerisationsreaktionen, kann das 1,3-Butadien zumindest teilweise entfernt werden. Wurde ein Crack-C₄-Schnitt als Rohstoff eingesetzt, bleibt in allen Fällen ein Kohlenwasserstoffgemisch (Raffinat I oder hydriertes Crack-C₄ (HCC₄)) zurück, das hauptsächlich die gesättigten Kohlenwasserstoffe, n-Butan und Isobutan und die Olefine Isobuten, 1-Buten und 2-Butene enthält.

Bevorzugt wird in dem erfindungsgemäßen Verfahren in einer zusätzlichen Reinigungsstufe, die einer oder mehreren der Verfahrensschritte a), b), c), d), e) oder f) vorgeschaltet wird, in den C₄-Kohlenwasserstoffströmen enthaltene mehrfach ungesättigten Kohlenwasserstoffe katalytisch selektiv hydriert. Besonders bevorzugt ist zumindest vor dem Verfahrensschritt a) oder c) und ganz besonders bevorzugt vor der Verfahrensstufe c) eine solche Reinigungsstufe vorgesehen, insbesondere wenn nicht ausgeschlossen werden kann, dass die eingesetzten technischen C₄-Kohlenwasserstoffströme mehrfach ungesättigte Kohlenwasserstoffe aufweisen.

Bei den mehrfach ungesättigten Kohlenwasserstoffen handelt es sich hauptsächlich um 1,3-Butadien; 1,2-Butadien, Butenin und 1-Butin sind, wenn überhaupt, in deutlich geringerer Menge enthalten. Die Hydrierung kann in einem ein- oder mehrstufigen Hydrierprozess in der Flüssigphase an einem Palladiumkontakt erfolgen. Zur Absenkung des Gehalts an 1,3-Butadien unter vorzugsweise 1000 wppm wird dabei in der letzten Stufe der Hydrierung mit Zusatz eines Moderators gearbeitet, der die Selektivität des Palladiumkontakts erhöht. Bevorzugt wird als Moderator Kohlenmonoxid eingesetzt, das in einem Anteil von 0,05 bis 100 Massen-ppm (wppm) zugesetzt wird. Der Gehalt an mehrfach ungesättigten Kohlenwasserstoffen sollte im Zulauf zu dieser Stufe unter 1 %, bevorzugt unter 0,5 %, betragen. In der Literatur ist diese Art der Selektivhydrierung von Restgehalten an 1,3-Butadien unter der Bezeichnung SHP (selective hydrogenation process) bekannt (vergleiche EP 0 081 041; Erdöl, Kohle, Erdgas, Petrochem. 1986, 39, 73).

Sind in den Isobuten-haltigen C₄-Strömen Mengen mehr als 1 % an mehrfach ungesättigten Kohlenwasserstoffe wie 1,3-Butadien enthalten, werden sie vorzugsweise in vorgeschalteten Hydrierungen umgesetzt. Diese Hydrierungen werden bevorzugt in der Flüssigphase an einem Palladiumkontakt durchgeführt. Je nach Gehalt an ungesättigten Kohlenwasserstoffen kann die Hydrierung in mehreren Stufen durchgeführt werden. Zur Umsetzung von Crack-C₄ aus einem Steam-Cracker mit einem Gehalt an 1,3-Butadien von typischerweise 38 bis 45 % hat sich eine zweistufige Ausführung der Hydrierung bewährt. Dabei können einzelne oder alle Stufen mit einer teilweisen Produktrückführung ausgestattet sein. Im Austrag sind so Konzentrationen an 1,3-Butadien kleiner 1 % erhältlich, so dass eine weitere Umsetzung in einer Selektivhydrierung (SHP) erfolgen kann.

Die in dem erfindungsgemäßen Verfahren eingesetzten Kohlenwasserstoffmischungen mit Isobuten und linearen Butenen weisen bevorzugt die folgenden Zusammensetzungen auf, wobei je nach Gehalt an ungesättigten Kohlenwasserstoffen eine Hydrierung oder Selektivhydrierung vor einem der Schritte a) bis d), vorzugsweise vor Schritt a) oder c) durchgeführt wird.

**Tabelle 1: Typische Zusammensetzungen von technischen Kohlenwasserstoffgemischen, die im erfindungsgemäßen Verfahren eingesetzt werden können.**

| | Steamcracker | | Steamcracker | | Katcracker | |
|---|---|---|---|---|---|---|
| Komponente | HCC₄ | HCC₄ / SHP | Raff. I | Raff. I / SHP | CC₄ | CC₄ / SHP |
| Isobutan [Massen-%] | 1-4,5 | 1-4,5 | 1,5 - 8 | 1,5 - 8 | 36-37 | 36 - 37 |
| n-Butan [Massen-%] | 5-8 | 5-8 | 6-15 | 6-15 | 12-14 | 12-14 |
| trans-Buten [Massen-%] | 18-21 | 18-21 | 7-10 | 7-10 | 11-13 | 11-13 |
| 1-Buten [Massen-%] | 35-45 | 35-45 | 15-35 | 15-35 | 11-13 | 11-13 |
| Isobuten [Massen-%] | 22 - 28 | 22 - 28 | 33 - 50 | 33 - 50 | 14 - 16 | 14 - 16 |
| cis-Buten [Massen-%] | 5-9 | 5-9 | 4-8 | 4-8 | 10-12 | 10-12 |
| 1,3-Butadien [wppm] | 500 - 8000 | 0 - 50 | 50 - 8000 | 0 - 50 | < 10000 | 0 - 50 |

| | | | | | | |
|---|---|---|---|---|---|---|
| Erläuterung - HCC₄: typisch für eine C₄ Mischung, die aus dem Crack-C₄ eines Steamcrackers (High Severity) nach der Hydrierung des 1,3-Butadiens ohne zusätzliche Moderation des Katalysators erhalten wird. - HCC₄ / SHP: Zusammensetzung HCC₄, bei dem Reste an 1,3-Butadien in einer SHP weiter reduziert wurden. - Raff. I (Raffinat I): typisch für eine C₄ Mischung, die aus dem Crack-C₄ eines Steamcrackers (High Severity) nach der Abtrennung des 1,3-Butadiens, beispielsweise durch eine NMP-Extraktivrektifikation, erhalten wird. - Raff. I / SHP: Zusammensetzung Raff. I, bei dem Reste an 1,3-Butadien in einer SHP weiter reduziert wurden. - CC₄: typische Zusammensetzung eines Crack-C₄, das aus einem Katcracker erhalten wird. - CC₄ / SHP: Zusammensetzung CC₄, bei dem Reste an 1,3-Butadien in einer SHP weiter reduziert wurden. | | | | | | |

Das Raffinat I bzw. HCC₄ ist, neben anderen, ein bevorzugt eingesetztes Isobuten-haltiges Kohlenwasserstoffgemisch im Rahmen dieser Erfindung. Da Anlagen zur Aufarbeitung von C₄-Kohlenwasserstoffen in der Regel als Strang (Verbund mehrerer Anlagen) aufgebaut sind, ist es jedoch möglich, dass das Raffinat I bzw. HCC₄ vor dem Eintritt in das erfindungsgemäße Verfahren eine oder mehrere andere Prozessstufe(n) durchläuft. Diese Prozessstufe(n) kann/können beispielsweise auch ein Verfahren bzw. Verfahrensschritt(e) sein, wie sie in den Ausführungsformen zum Verfahrensschritt a) beschrieben worden sind. In dem erfindungsgemäßen Verfahren einsetzbare C₄-Kohlenwasserstoffgemische können auch solche sein, wie sie aus Verfahren gemäß den Ausführungsformen der Verfahrensschritt a) und anschließender Trennung gemäß Verfahrensschritt b) erhalten werden. Insbesondere können auch solche Gemische eingesetzt werden, wie sie bei der Herstellung von tert.-Butanol (TBA) aus Isobuten nach Abtrennung des TBA erhalten werden. Auf diese Weise kann jeweils ein individuell angepasstes Gesamtkonzept zur Aufarbeitung mit dem entsprechenden Produktportfolio realisiert werden.

Typische Verfahren, die den erfindungsgemäßen Verfahren vorgelagert sein können, sind Wasserwäschen, Reinigungsverfahren in Adsorbern, Trocknungsverfahren und Destillationen.

### Wasserwäsche

Durch eine Wasserwäsche können hydrophile Komponenten aus dem einzusetzenden technischen Kohlenwasserstoffgemisch, enthaltend Isobuten und lineare Butene, ganz oder teilweise entfernt werden, beispielsweise Stickstoffkomponenten. Beispiele für Stickstoffkomponenten sind Acetonitil oder N-Methylpyrrolidon (die z. B. aus einer 1,3-Butadien-Extraktivdestillation stammen können). Auch Sauerstoffverbindungen (z. B. Aceton aus FCC) können zum Teil über eine Wasserwäsche entfernt werden. Der Isobuten-haltige Kohlenwasserstoffstrom ist nach einer Wasserwäsche mit Wasser gesättigt. Um eine Zweiphasigkeit in den nachfolgenden Prozessschritten im Reaktor zu vermeiden, sollte dort die Reaktionstemperatur um ca. 10 °C über der Temperatur der Wasserwäsche liegen.

### Adsorber

Adsorber werden eingesetzt, um Verunreinigungen zu entfernen. Dies kann beispielsweise vorteilhaft sein, wenn in einem der Prozessschritte Edelmetallkatalysatoren zum Einsatz kommen. Oftmals werden Stickstoff oder Schwefelverbindungen über vorgeschaltete Adsorber entfernt. Beispiele für Adsorptionsmittel sind Aluminiumoxide, Molekularsiebe, Zeolithe, Aktivkohle, mit Metallen imprägnierte Tonerden. Adsorptionsmittel werden von diversen Firmen vertrieben, beispielsweise der Firma Alcoa (Selexsorb^{®}).

### Trocknung

Im Isobuten-haltigen Kohlenwasserstoffgemisch gegebenenfalls enthaltenes Wasser, das beispielsweise aus der Wasserwäsche stammen kann, kann durch bekannte Verfahren zur Trocknung entfernt werden. Geeignete Verfahren sind beispielsweise die destillative Abtrennung des Wassers als Azeotrop. Dabei kann oftmals ein Azeotrop mit enthaltenen C₄-Kohlenwasserstoffen ausgenutzt werden oder es können Schleppmittel zugesetzt werden.

Die Trocknung des Kohlenwasserstoffgemisches kann aus diversen Gründen vorteilhaft sein, beispielsweise zur Verringerung der Bildung von Alkoholen (hauptsächlich tert.-Butylalkohol) in Schritt a) oder zur Vermeidung von technischen Problemen durch Abscheidung von Wasser oder zur Vermeidung von Eisbildung bei niedrigen Temperaturen (z. B. bei der Zwischenlagerung).

### Destillation

Destillationsschritte können beispielsweise genutzt werden, um Verunreinigungen abzutrennen (beispielsweise Leichtsieder wie C₃-Kohlenwasserstoffe, Schwersieder wie C₅-Kohlenwasserstoffe) oder um Fraktionen mit unterschiedlichen Isobutenkonzentrationen zu erhalten. Dies kann sowohl direkt mit dem Raffinat I bzw. dem HCC₄ erfolgen oder nachdem eine oder mehrere andere Prozessstufe(n) durchlaufen wurde(n). Durch direkte Destillation des Raffinats I bzw. des HCC₄ ist beispielsweise eine Trennung in eine an 2-Butenen und n-Butan verarmte, Isobuten reichere Fraktion möglich.

Je nach Zusammensetzung des einzusetzenden technischen Kohlenwasserstoffgemisches und/oder nach den Reinheiten der Zielprodukte kann das technische Kohlenwasserstoffgemisch also direkt in den Schritt a) des erfindungsgemäßen Verfahrens oder aber erst nach einer Vorbehandlung durch eines oder mehrere der vorgenannten Verfahren eingesetzt werden.

Mit dem Verfahren lässt sich auf einfache Weise ETBE bzw. eine Zusammensetzung, die ETBE enthält, herstellen, welche/s als Sumpfprodukt der Reaktivdestillationen oder der Destillationen gemäß den Schritten b) und e), bevorzugt als Sumpfprodukt des Schrittes b) erhalten werden/wird.

Eine ETBE aufweisende Zusammensetzung, die z. B. mit dem Verfahren hergestellt werden kann und die bevorzugt als Sumpfprodukt II im Verfahrensschritt b) erhalten wird, enthält größer-gleich 90 Massenanteile ETBE, 0 bis 7 Massenanteile, vorzugsweise 0 bis 5 Massenanteile Ethanol, 0 bis 3 Massenanteile, vorzugsweise 0 bis 2,5 Massenanteile tert.-Butanol, kleiner-gleich 2 Massenanteile, vorzugsweise kleiner-gleich 1,5 Massenanteile Kohlenwasserstoffe mit einer Anzahl an Kohlenstoffatomen größer-gleich 5 und kleiner-gleich 1 Massenanteil Kohlenwasserstoffe mit einer Anzahl an Kohlenstoffatomen von 4. Eine solche Zusammensetzung kann z. B. durch das erfindungsgemäße Verfahren erhalten werden. Es kann vorteilhaft sein, wenn die erfindungsgemäße Zusammensetzung maximal von 1 x 10⁻⁴ bis 1000 x 10⁻⁴ Massenanteile an Diethylether aufweist. Die erfindungsgemäße Zusammensetzung weist vorzugsweise kein heterogenes Wasser auf. Besonders bevorzugt weist die erfindungsgemäße Zusammensetzung weniger als 0,05 Massenanteile Wasser auf. Besonders bevorzugt weist die erfindungsgemäße Zusammensetzung größer-gleich 90 Massenanteile, insbesondere von 90 bis 97,5 Massenanteile ETBE, von 1 bis 5 Massenanteile Ethanol, von 0,5 bis 1 Massenanteile tert.-Butanol, von 0,5 bis 1 Massenanteile Kohlenwasserstoffe mit einer Anzahl an Kohlenstoffatomen größer-gleich 5, von 0,1 bis 0,5 Massenanteile Kohlenwasserstoffe mit einer Anzahl an Kohlenstoffatomen von 4 und von 0 bis 0,05, insbesondere von 0,0001 bis 0,01 Massenteile an Wasser auf. Die für die erfindungsgemäßen Zusammensetzungen angegebenen Massenanteile sind besonders bevorzugt Massen-%.

Das ETBE kann als Kraftstoff oder Kraftstoffzusatz eingesetzt werden. Da zur Herstellung des ETBE Bioethanol, also Ethanol, der aus nachwachsenden Rohstoffen erhalten wurde, eingesetzt werden kann, kann durch das Verfahren ein Beitrag zur Schonung der fossilen Brennstoffreserven geleistet werden. Neben ETBE fällt bei dem Verfahren ein 1-Buten haltiger Strom an, der zu 1-Buten, welches z. B. als Comonomer bei der Herstellung von Polymeren eingesetzt wird, aufgearbeitet werden kann.

### Beschreibung der Figuren

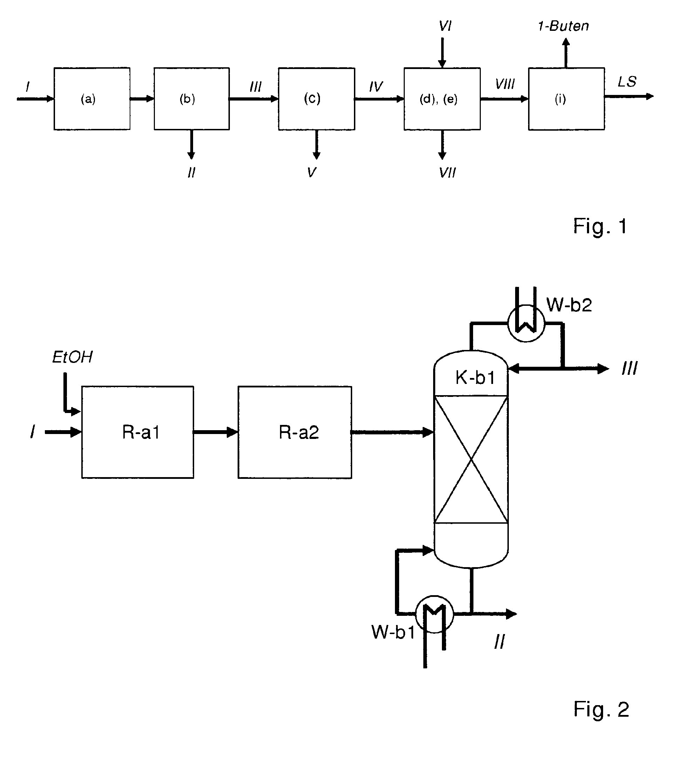

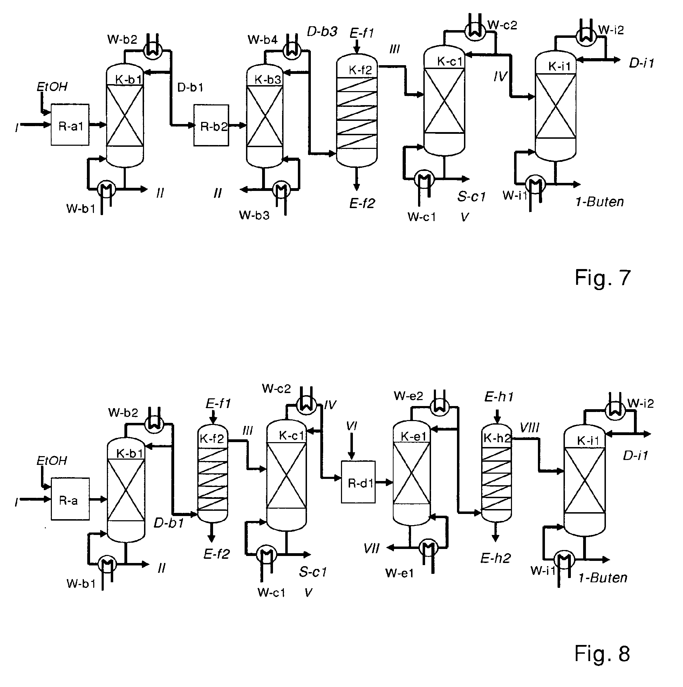

An Hand der Figuren Fig. 1 bis Fig. 5 und Fig. 8 wird das erfindungsgemäße Verfahren nachfolgend näher erläutert, ohne dass das Verfahren auf die dort beispielhaft abgebildeten Ausführungsarten beschränkt sein soll. In den Figuren Fig. 6 und Fig. 7 sind Vergleichsvarianten dargestellt. In den schematischen Darstellungen sind nur die wesentlichen Stufen dargestellt. Auf die Darstellung von verfahrenstechnisch üblichen Strömen, wie z. B. Kühlwasserströmen, Kreislaufströmen oder Rückführungen, und/oder üblichen Apparaturen, wie z. B. Wärmetauschern oder Abscheidern, wurde teilweise zu Gunsten einer besseren Übersicht verzichtet.
Fig. 1
   Bei dem in Fig.1 schematisch dargestellten Verfahren wird in die Stufe (a) eine technische Mischung von C₄-Kohlenwasserstoffen eingebracht. In der Stufe (a) erfolgt eine teilweise Umsetzung des in der technischen Mischung enthaltenen Isobutens mit Ethanol. Das Produkt der Stufe (a) wird in die Trennstufe (b) überführt, in der nicht umgesetzte C₄-Kohlenwasserstoffe III und gegebenenfalls im Überschuss vorhandenes Ethanol vom ETBE II vorzugsweise thermisch abgetrennt werden. Die nicht umgesetzten C₄-Kohlenwasserstoffe III werden in eine Stufe (c) überführt, die z. B. durch eine einfache Destillationskolonne realisiert sein kann. In dieser Kolonne wird der Strom III in eine Fraktion IV, die Isobuten, Isobutan und 1-Buten aufweist, und eine Isobuten-freie oder nahezu Isobuten-freie Fraktion V, die 2-Butene und n-Butane aufweist, getrennt. Die Fraktion IV wird in die zweite Umsetzungsstufe (d) überführt, in welcher das Isobuten mit Ethanol erneut zu ETBE umgesetzt wird. In einer anschließenden Trennstufe (e) wird der ETBE VII von nicht umgesetzten Kohlenwasserstoffen VIII getrennt. Diese Kohlenwasserstoffe VIII werden in die Stufe (i) überführt, in welcher das 1-Buten destillativ von den restlichen Kohlenwasserstoffen getrennt wird.
Fig. 2
   In Fig. 2 ist schematisch eine mögliche Ausführungsform der Verfahrensschritte a) und b) dargestellt. Das technische Gemisch I wird zunächst in einen ersten Veretherungsreaktor R-a1 gefahren. Das Produkt aus dem ersten Reaktor wird in einen zweiten Veretherungsreaktor R-a2 gefahren (Fahrweise mit gleicher oder unterschiedlicher Temperatur möglich). Der Austrag aus dem zweiten Veretherungsreaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Kopfprodukt wird der Strom III abgenommen, der nicht umgesetzte C₄-Kohlenwasserstoffe aufweist, und als Sumpfprodukt wird das Produkt II aus der Umsetzung des Isobutens erhalten, welches hauptsächlich aus ETBE besteht.
Fig. 3

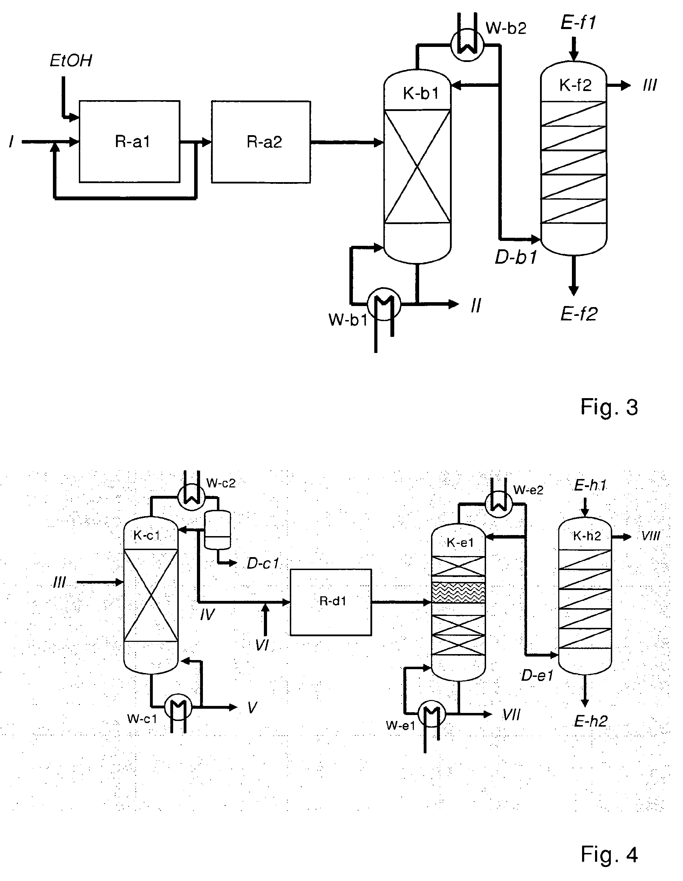

   In Fig. 3 ist schematisch eine weitere mögliche Ausführungsform der Verfahrensschritte a), b) und f) dargestellt. Das technische Gemisch I wird in den ersten Reaktor R-a1 einer Kaskade von zwei Reaktoren eingespeist, in den auch Ethanol eingespeist wird. Der Reaktor R-a1 weist eine Rückführung auf, mit der ein Teil des Reaktoraustrags in den Zustrom zum Reaktor zurückgefahren werden kann. Der andere Teil des Reaktoraustrags aus dem ersten Reaktor wird in den zweiten Reaktor R-a2 gefahren. Der Austrag aus dem zweiten Reaktor wird in eine Destillationskolonne K-b1 überführt, die mit einem Kondensator W-b2 für das Kopfprodukt und einem Sumpfverdampfer W-b1 ausgestattet ist. Ein Teil des Kopfproduktes wird als Rücklauf in die Kolonne zurückgefahren. Als Sumpfprodukt wird das Produkt II, hauptsächlich ETBE und ggf. Restmengen an Ethanol erhalten. Als Kopfprodukt wird der Strom D-b1 abgenommen, der nicht umgesetzte Kohlenwasserstoffe und gegebenenfalls noch Ethanol aufweist. Enthält der Strom Ethanol kann dieser Strom unten in eine Extraktionskolonne K-f2 gefahren werden, in die im Gegenstrom ein Extraktionsmittel, wie z. B. Wasser, über den am Kopf befindlichen Zulauf E-f1 eingespeist wird, welches über den Ablauf E-f2 am Sumpf der Kolonne entnommen wird. Am Kopf der Kolonne wird als Produkt der Extraktion der Strom aus in Stufe (a) nicht umgesetzten Kohlenwasserstoffen III erhalten.
Fig. 4
   In Fig. 4 wird eine mögliche Ausführungsform der Stufen c), d), e) und h) dargestellt. In eine Destillationskolonne K-c1, die mit einem Sumpfverdampfer W-c1 und am Kopf mit einem Kondensator W-c2 und einem Dekanter ausgerüstet ist, wird der Kohlenwasserstoffstrom III aus Stufe b) eingespeist und in eine (nahezu) Isobuten-freie, 2-Butene und n-Butane aufweisende Fraktion V, die am Sumpf der Kolonne abgenommen wird, und eine Isobuten und 1-Buten aufweisende, nahezu n-Butan und 2-Butene freie Fraktion IV, die gegebenenfalls in einem Dekanter von einer wässrigen Phase D-c1 getrennt wird, aufgetrennt. Ein Teil des um den wässrigen Anteil verminderten Kopfprodukts kann als Rücklauf in die Kolonne zurückgefahren werden. Die Fraktion IV wird in den Reaktor R-d1 überführt, in welchen außerdem Ethanol eingespeist wird und in dem das in der Fraktion IV enthaltenes Isobuten zu ETBE umgesetzt wird (Stufe d)). Der Austrag aus dem Reaktor R-d1 wird in eine Kolonne K-e1 eingespeist, die als einfache Destillationskolonne oder wie hier dargestellt als Reaktivkolonne ausgeführt sein kann. Die Zuführung des Austrags aus dem Reaktor erfolgt in die Reaktivdestillationskolonne K-e1 vorzugsweise unterhalb der reaktiven Packung. Die Kolonne K-e1 ist mit einem Sumpfverdampfer W-e1 und einem Kondensator W-e2 für das Kopfprodukt ausgerüstet. Als Sumpfprodukt der Kolonne K-e1 wird ein ETBE enthaltender Strom erhalten. Das Kopfprodukt D-e1 kann teilweise als Rücklauf in die Kolonne zurückgefahren werden. Der andere Teil wird in die Extraktionskolonne K-h2 überführt, in die im Gegenstrom ein Extraktionsmittel, wie z. B. Wasser, über den am Kopf befindlichen Zulauf E-h1 eingespeist wird, welches über den Ablauf E-h2 am Sumpf der Kolonne entnommen wird. Am Kopf der Kolonne wird als Produkt der Extraktion der Strom aus in Stufe d) und gegebenenfalls e) nicht umgesetzten Kohlenwasserstoffen VIII erhalten.
Fig. 5

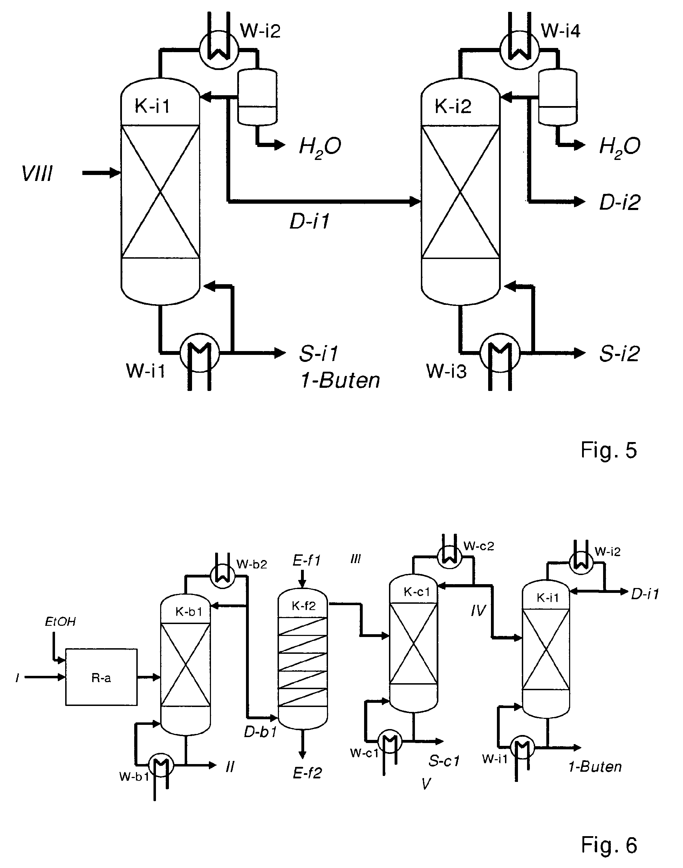

   In Fig. 5 ist schematisch eine mögliche Ausführungsform des Verfahrensschrittes i) dargestellt. Der Kohlenwasserstoffstrom VIII aus Stufe e) oder h) wird in eine Destillationskolonne K-i1 eingespeist. Die Kolonne K-i1 ist mit einem Sumpfverdampfer W-i1 und einem Kondensator W-i2 für das Kopfprodukt ausgerüstet. Als Sumpfprodukt S-i1 der Kolonne K-i1 wird 1-Buten erhalten. Das Kopfprodukt D-i1, von dem gegebenenfalls in einem Dekanter Wasser abgetrennt wird, wird teilweise als Rücklauf in die Kolonne zurückgefahren. Der andere Teil des Kopfproduktes D-i1 wird in die Destillationskolonne K-i2 überführt. Auch diese Kolonne K-i2 ist mit einem Sumpfverdampfer W-i3 und einem Kondensator W-i4 für das Kopfprodukt ausgerüstet. Als Sumpfprodukt S-i2 der Kolonne K-i2 wird Isobutan erhalten. Das Kopfprodukt D-i2, von dem gegebenenfalls in einem Dekanter Wasser abgetrennt wird, wird teilweise als Rücklauf in die Kolonne zurückgefahren. Der andere Teil des Kopfproduktes D-i2, der überwiegend aus Leichtsiedern besteht, kann einer weiteren Nutzung oder einer thermischen Verwertung zugeführt werden.
   Das bei dieser Aufarbeitung erhaltene Isobutan (Strom S-i2) kann noch Anteile an ungesättigten Komponenten, hauptsächlich 1-Buten, enthalten. Diese können in einer nachgeschalteten Hydrierung zu den entsprechenden Alkanen hydriert werde. Diese Hydrierung erfolgt nach bekannten Verfahren der Technik, bevorzugt in der flüssigen Phase an einem Palladiumkatalysator. Optional kann diese Hydrierung auch vor der Kolonne K-i2 erfolgen; der Strom D-i1 wird in diesem Fall erst der Hydrierung (in Fig. 5 nicht dargestellt) und anschließend der K-i2 zugeführt.
Fig. 6
   In dieser Figur ist die im Vergleichsbeispiel berechnete Variante A eines einstufigen Prozesses dargestellt. Bei dieser Variante werden die Stufen (a) und (b) in einer wie in Fig. 3 dargestellten Verschaltung durchgeführt, wobei ein beliebiges Reaktorsystem R-a an Stelle der Reaktoren R-a1 und R-a2 vorhanden ist. Das am Kopf der Extraktionskolonne K-f2 erhaltene Produkt III wird in die Destillationskolonne K-c1 überführt, in welcher Isobutan, Isobuten und 1-Buten über Kopf abgetrennt werden. Als Sumpfprodukt S-c1 wird eine (nahezu) Isobuten-freie, 2-Butene und n-Butane aufweisende Fraktion V erhalten. Das Destillat IV der Kolonne K-c1 wird direkt in eine weitere Kolonne K-i1 geführt, in welcher es in ein 1-Buten enthaltendes Sumpfprodukt und ein Isobutan und/oder Leichtsieder aufweisendes Kopfprodukt getrennt wird. Als Sumpfprodukt wird eine 1-Buten-reiche Fraktion erhalten, die allerdings den größten Teil des in R-a nicht umgesetzten Isobutens enthält.
Fig. 7
   In dieser Figur ist die im Vergleichsbeispiel berechnete Variante B eines zweistufigen Prozesses dargestellt. Bei dieser Variante werden die Stufen (a) und (b) in einer wie in Fig. 2 dargestellten Verschaltung durchgeführt, wobei ein beliebiges Reaktorsystem R-a1 an Stelle der Reaktoren R-a1 und R-a2 vorhanden ist. Das aus der Kolonne K-b1 erhaltene Destillat D-b1 wird direkt in einen zweiten Reaktor R-b2 gefahren, in dem das im Destillat D-b1 vorhandene restliche Isobuten mit dem vorhandenen und gegebenenfalls zugegebenem Ethanol umgesetzt wird. Das Reaktionsprodukt aus dem Reaktor R-b2 wird in eine Kolonne K-b3 gefahren, in der der in R-b2 gebildete ETBE vom restlichen C₄-Kohlenwasserstoffstrom D-b3 als Sumpfprodukt II abgetrennt wird. Die weitere Aufarbeitung des Destillats D-b3 erfolgt wie in Fig. 6 für das Destillat D-b1 dargestellt.
Fig. 8
   Zum besseren Vergleich der Verschaltung gemäß der Ausführungsform des erfindungsgemäßen Verfahrens, wie sie im Beispiel in Variante C eingesetzt wurde, mit den Vergleichsvarianten A und B gemäß den Fig. 6 und 7 ist in Fig. 8 schematisch eine Verschaltung dargestellt, bei der sowohl in Stufe (a) als auch in Stufe (d) ein Veretherungsschritt durchgeführt wird. Die Stufen (a) und (b) werden in einer wie in Fig. 3 dargestellten Verschaltung durchgeführt, wobei ein Reaktorsystem R-a an Stelle der Reaktoren R-a1 und R-a2 vorhanden ist. Die Stufen (c), (d) und (e) werden wie in Fig. 4 beschrieben durchgeführt. Das Produkt VIII, welches aus der Extraktionskolonne K-h2 erhalten wird, wird in die Destillationskolonne K-i1 geführt, in welcher es in ein 1-Buten enthaltendes Sumpfprodukt und ein Isobutan und/oder Leichtsieder aufweisendes Kopfprodukt getrennt wird.
   Die folgenden Beispiele sollen die Erfindung erläutern.

### Beispiele

Die nachfolgenden Beispielrechnungen wurden mit dem Simulationsprogramm ASPEN Plus durchgeführt. Um transparente, reproduzierbare Daten zu erzeugen, wurden nur allgemein zugängliche Stoffdaten eingesetzt. Auf den Einsatz kinetischer Ansätze wurde bewusst verzichtet. Außerdem wurde bei allen Varianten auf den Einsatz einer Reaktivdestillation verzichtet. Durch diese Vereinfachungen ist es dem Fachmann leicht möglich, die Berechnungen nachzuvollziehen. Die eingesetzten Methoden besitzen zwar keine ausreichende Genauigkeit für die Auslegung technischer Anlagen, die qualitativen Unterschiede der Schaltungen werden aber korrekt erfasst. In allen gezeigten Varianten kann der Isobutenumsatz durch Einsatz einer oder mehrerer Reaktivdestillation(en) erhöht werden.

Die Reaktoren und ETBE-Kolonnen wurden mit der Property-Methode "UNIFAC-DMD" berechnet. Für die Berechnung der C₄-Kolonnen wurde mit der Property-Methode "Peng-Robinson" eine Zustandsgleichung eingesetzt. Folgende Annahmen wurden getroffen:
- Alle Reaktoren erreichen bei 50 °C vollständig das mit UNIFAC berechnete Gleichgewicht.
- Die ETBE-Kolonnen wurden mit einem Rücklaufverhältnis von 0,8 berechnet. Für die C₄-Kolonnen wurden die gewünschten Reinheiten der Zielfraktionen als Vorgaben für die Simulationen eingesetzt.
- In den ETBE-Kolonnen wurde über Kopf ein C₄-/Ethanol-Azeotrop abgetrennt. Das EtOH wurde in Extraktoren mit Wasser ausgewaschen, die als einfache Komponenten-Splitter modelliert wurden.
- Das aus den Extraktoren erhaltene EtOH-Wasser-Gemisch wurde in einer weiteren Kolonne K-EtOH destillativ aufgearbeitet, die in den Schaltbildern nicht dargestellt wurde. Beide Produkte der K-EtOH wurden - gegebenenfalls nach einer geeigneten Trocknung des Ethanols - in den Prozess rezirkuliert.

Den berechneten Beispielen wurde ein Rohstoffmix aus typischen, auf dem Markt erhältlichen C₄-Rohstoffen zugrundegelegt. Der Rohstoffstrom von 10 t/h enthielt 28 Massen-% Isobuten und 35 Massen-% 1-Buten.

Aus diesem Strom sollte das Isobuten möglichst vollständig in ein ETBE-Produkt mit einem Ethanol-Gehalt von ca. 5 Massen-% umgesetzt werden. Ferner sollte das Isobuten durch die ETBE-Synthese chemisch soweit abgetrennt werden, dass ein 1-Buten-Produkt in einer Menge von 3 t/h mit einer Reinheit größer 99,5 % hergestellt werden kann. Dies entspricht einer 1-Buten-Ausbeute von ca. 85 %. Im 1-Buten Produkt sollten maximal 2000 ppm Isobuten vorliegen. In Tabelle 2 wurde die Zusammensetzung des C₄-Rohstoffstroms der gewünschten Spezifikation des als Nebenprodukt anfallenden 1-Butens gegenübergestellt. Die eingesetzte Menge an Ethanol betrug 2426 bis 2545 kg/h (d. h. 5 bis 11 %-iger molarer Überschuss, vgl. unten) mit einem Wassergehalt von 1 Massen-%.

**Tabelle 2: Zusammensetzung des C₄-Rohstoffstroms und 1-Buten Spezifikation (in Massen-%)**

| Komponente | C₄-Feed | | 1-Buten | |
|---|---|---|---|---|
| | [kg/h] | [%] | [kg/h] | [%] |
| Isobutan | 500 | 5,0 | 3 | 0,2 |
| 1-Buten | 3500 | 35,0 | 2990 | 99,5 |
| cis-2-Buten | 1400 | 14,0 | | 0,0 |
| trans-2-Buten | 800 | 8,0 | | 0,0 |
| Isobuten | 2800 | 28,0 | 6 | 0,2 |
| n-Butan | 1000 | 10,0 | 1 | 0,1 |
| Summe | 10000 | 100 | 3000 | 100 |

Nachfolgend wurden drei zur Lösung der Aufgabenstellung unterschiedlich gut geeignete Verfahrensvarianten berechnet.

Die einfachste Variante A war ein einstufiger Prozess, der als Vergleich dienen sollte. Nach Fig. 6 wurden Ethanol und Isobuten in einer Reaktionsstufe R-a bis ins Gleichgewicht umgesetzt. In der Destillationsstufe K-b1 wurde das ETBE als Sumpfprodukt (II) abgetrennt. Die Kolonne hatte 50 theoretische Stufen und wurde bei einem Rücklaufverhältnis von 0,8 betrieben. Das Destillat dieser Kolonne war ein C₄/EtOH-Azeotrop, aus dem das Ethanol z. B. mit Wasser in einer Extraktionskolonne K-f2 ausgewaschen wurde. Das Raffinat der Extraktionskolonne K-f2 wurde einer C₄-Kolonne K-c1 zugeführt, in der Isobutan, Isobuten und 1-Buten über Kopf abgetrennt wurden. Das Destillat IV der K-c1 wurde direkt in eine weitere Kolonne K-i1 geleitet, in der vornehmlich Isobutan über Kopf abgetrennt wurde. Als Sumpfprodukt wurde eine 1-Buten-reiche Fraktion erhalten, die den größten Teil des in R-a nicht umgesetzten Isobutens enthielt.

Das mit Variante A dargestellte 1-Buten enthielt 1,91% Isobuten, siehe Tabelle 3, und erreichte somit die Zielvorgabe von 2000 ppm nicht. Außerdem war der molare Ethanol-Überschuß im Zulauf des R-a bei Variante A auf etwa 5 % (im Beispiel 2426 kg/h) begrenzt, da das Überschuß-EtOH nur im Kopf der Kolonne K-b1 als Azeotrop abgetrennt werden konnte. Damit wurden im ETBE-Produkt bereits 5,2 % EtOH gefunden. Es wurden 5113 kg/h ETBE-Produkt mit einer Reinheit von 93,6 % gebildet.

Eine Prozessverbesserung wurde als Variante B untersucht und ist in Fig. 7 dargestellt. Um das Gleichgewicht weiter in Richtung ETBE zu treiben, wurde hinter die Kolonne K-b1 ein weiterer Reaktor R-b2 geschaltet, der das Rest-Isobuten mit dem in K-b1 als Azeotrop D-b1 über Kopf abgetrennten Ethanol umsetzte. Dem Reaktor konnte zur Erhöhung des Umsatzes ein weiterer EtOH-Strom von 200 kg/h zugeführt werden. Das gegenüber Variante A zusätzlich gebildete ETBE wurde in einer weiteren C₄-/ETBE-Destillation K-b3 abgetrennt. Dabei musste der gesamte C₄-Strom ein zweites Mal über Kopf destilliert werden, der Energiebedarf der K-b3 war also nahezu gleich groß wie der der K-b1. Anschließend wurde die Extraktion K-f2 und die 1-Buten-Destillation K-c1 und K-i1 wie in Variante A durchfahren. Nun erreichte das 1-Buten-Produkt mit weniger als 2000 ppm Isobuten knapp die geforderte Produktspezifikation. Wegen des höheren Isobuten-Umsatzes wurden 5340 kg/h ETBE-Produkt (Variante A: 5113 kg/h) mit einer Reinheit von 93,7 % gebildet. Der Gesamtenergiebedarf der Anlage war gegenüber Variante A allerdings um etwa 13 % höher.

Im erfindungsgemäßen Verfahren Variante C nach Fig. 8 wurde eine zweite Reaktions-, Destillations- und Extraktionsstufe R-d1, K-e1 und K-h2, mit einer zweiten EtOH-Zufuhr wie unter Variante B, zwischen die beiden C₄-Kolonnen K-c1 und K-i1 geschaltet. Dies hatte den Vorteil, dass der größere Teile des Zulaufs III der K-c1 als Sumpfprodukt V anfiel und nur der Teil des C₄-Stromes ein zweites Mal destilliert wurde, der in K-i1 zu reinem 1-Buten aufgearbeitet werden sollte. Im vorliegenden Fall war der Energiebedarf der K-f2 bei Variante B mehr als doppelt so hoch, wie der Energiebedarf der K-e1 bei Variante C. Nachteilig an Variante C war, dass eine zweite Extraktionskolonne K-h2 vorgesehen werden musste. Da der Durchsatz durch R-d1, K-e1 und K-h2 aber weniger als die Hälfte des Durchsatzes durch K-b3 bei Variante B betrug, fielen die Gesamtinvestitionskosten entsprechend niedriger aus.

Die Isobutenmenge im 1-Buten-Produkt lagen im berechneten Beispiel bei deutlich weniger als 2000 ppm. Die Menge und Zusammensetzung des erhaltenen ETBE-Produktes war bei Rückführung des Stroms VII in die ersten Reaktionsstufe a) sehr ähnlich zu Variante B.

Tabelle 3 zeigt die erreichten Umsätze der drei Varianten. Während Variante A die geforderte Qualität des 1-Buten-Produktes klar verfehlte, wurde in den beiden zweistufigen Verfahren B und C ein spezifikationsgerechtes Nebenprodukt berechnet. Der erhöhte Isobuten-Umsatz wurde bei beiden Varianten durch destillative Abtrennung des Reaktionsproduktes ETBE vor einer zweiten Reaktionsstufe und damit durch erhöhten Energieeinsatz erreicht. Durch die erfindungsgemäße Anordnung der zweiten Reaktionsstufe in Variante C zwischen den beiden C₄-Kolonnen K-c1 und K-i1 wurde die Menge des zusätzlich zu destillierenden Stromes jedoch auf weniger als die Hälfte reduziert. Dies führte zu deutlichen Einsparungen bei Energiebedarf und Investment.

**Tabelle 3: Umsätze und 1-Buten-Qualitäten der drei Varianten**

| | Variante A, einstufig | Variante B, zweistufig | Variante C, zweistufig |
|---|---|---|---|
| Isobuten im Feed [kg/h] | 2800 | 2800 | 2800 |
| Ethanol-Feed (netto) [kg/h] | 2426 | 2545 | 2548 |
| Isobuten nach Stufe 1 [kg/h] | 133,1 | 149,1 | 149,1 |
| Umsatz Stufe 1 [%] | 95,2 | 94,7 | 94,7 |
| Isobuten nach Stufe 2 [kg/h] | | 6,6 | 5,6 |
| Umsatz Stufe 2 [%] | | 95,6 | 96,2 |
| Umsatz Gesamt [%] | 95,2 | 99,8 | 99,8 |
| Isobuten im 1-Buten [ppm] | 19130 | 1957 | 1833 |
| Reinheit 1-Buten [%] | 97,8 | 99,5 | 99,5 |

In Tabelle 4 sind die berechneten Energiebedarfszahlen aller drei Varianten zusammengestellt. In allen Schaltungen war der Energiebedarf der Kolonnen K-b1, K-c1 und K-i1 nahezu identisch. Variante A hatte zwar den niedrigsten Gesamtenergiebedarf, die Produktspezifikation wurde jedoch verfehlt. Bei Variante B musste im ETBE-Teil die doppelte C₄-Menge über Kopf destilliert werden, ihr Energiebedarf lag daher um 13 % höher als bei Variante A. Demgegenüber zeigte Variante C einen Weg, den geforderten Isobutenumsatz bei nur etwa 6 % erhöhtem Energiebedarf zu erreichen.

**Tabelle 4: Energiebedarf der drei Varianten**

| | Variante A, einstufig | Variante B, zweistufig | Variante C, zweistufig |
|---|---|---|---|
| Q K-b1 [kW] | 1284 | 1287 | 1287 |
| Q K-EtOH [kW] | 126 | 122 | 155 |
| Q K-b3 [kW] | | 1267 | |
| Q K-c1 [kW] | 3510 | 3501 | 3580 |
| Q K-e1 [kW] | | | 593 |
| Q K-i1 [kW] | 3813 | 3680 | 3674 |
| Q Gesamt [kW] | 8733 | 9857 | 9289 |
| Mehrbedarf ΔQ gegenüber Variante A [%] | 0 | 12,9 | 6,4 |

In Tabelle 5 sind die berechneten ETBE-Qualitäten, wie sie mit den Varianten A, B und C erhalten werden können, dargestellt

**Tabelle 5: ETBE-Qualitäten der drei Varianten:**

| | Variante A, einstufig | Variante B, zweistufig | Variante C, zweistufig |
|---|---|---|---|
| Ethanol-Zulauf (gesamt) [kg/h] | 2426 | 2545 | 2548 |
| molarer EtOH-Überschuss in % | 5,5 | 10,7 | 10,8 |
| EtOH im ETBE [Massen-%] | 5,2 | 5,0 | 5,1 |
| ETBE im ETBE [Massen-%] | 93,6 | 93,8 | 93,8 |
| ETBE-Menge [kg/h] | 5113 | 5340 | 5318 |

Tabelle 5 kann entnommen werden, dass mit den Varianten A, B und C ähnliche Qualitäten an ETBE gewonnen werden konnten, wobei die Qualität des ETBE gemäß Variante A bezüglich des Ethanol- und des ETBE-Gehalts jeweils höher bzw. niedriger als bei den Varianten B und C und damit schlechter war. Da mit der Variante A, wie Tabelle 3 entnommen werden kann, zusätzlich auch nur ein relativ verunreinigtes 1-Buten als Nebenprodukt erhalten werden konnte, sind die Varianten B oder C trotz des höheren Energiebedarfs zu bevorzugen. Den in Tabelle 4 aufgelisteten Ergebnissen kann entnommen werden, dass die erfindungsgemäße Variante C einen deutlich geringeren Wärmebedarf als die Variante B aufwies und damit in Bezug auf die Qualität von ETBE und 1-Buten die energetisch günstigste Variante darstellt.

Die Bezeichnungen in den Figuren Fig. 1 bis Fig. 8 haben folgende Bedeutungen:
- (a): Teilumsetzung Isobuten zu Produkten
- (b): Auftrennung des Produkts aus (a) in eine ETBE aufweisende Fraktion II und C₄-Kohlenwasserstoffe III
- (c): destillative Trennung von III in IV und V
- (d): Veretherung von Isobuten mit Ethanol VI
- (e): Abtrennung eines ETBE-haltigen Stroms VII
- (i): Abtrennung 1-Buten

- I: technische Mischung von C₄-Kohlenwasserstoffen
- II: ETBE aufweisende Fraktion
- III: verbleibende C₄-Kohlenwasserstoffe
- IV: 1-Buten und Isobuten-haltige Fraktion
- V: Isobuten-freie, 2-Butene und n-Butane aufweisende Fraktion
- VI: Ethanol
- VII: ETBE-haltiger Strom
- VIII: C₄-Kohlenwasserstoffe aus Stufe e) bzw. h)

- D-b1: Destillat der K-b 1
- D-b3: Destillat der K-b3
- D-c1: wässrige Phase aus Dekanter der K-c1
- D-e1: Kopfprodukt von K-e1
- D-i1: Destillat K-i1, organische Phase
- D-i2: Leichtsieder
- E-f1: Zulauf Extraktionsmittel
- E-f2: Ablauf Extraktionsmittel
- E-h1: Zulauf Extraktionsmittel
- E-h2: Ablauf Extraktionsmittel
- K-b1: Destillation
- K-b3: Destillation
- K-c1: Kolonne zur Trennung der C₄-Kohlenwasserstoffe
- K-e1: Kolonne zur Abtrennung des Ethers
- K-f2: Extraktionskolonne
- K-h2: Extraktionskolonne
- K-i1: Kolonne zur 1-Buten Abtrennung
- K-i2: Kolonne zur Isobutan-Abtrennung
- R-a: Reaktor
- R-a1: Reaktor
- R-a2: Reaktor
- R-b2: Reaktor Veretherung (Vergleichsbeispiel)
- R-d1: Reaktor Veretherung
- S-i1: 1-Buten
- S-i2: Isobutan
- W-b1: Sumpfverdampfer
- W-b2: Kondensator
- W-b3: Sumpfverdampfer
- W-b4: Kondensator
- W-c1: Sumpfverdampfer
- W-c2: Kondensator
- W-e1: Sumpfverdampfer
- W-e2: Kondensator
- W-i1: Sumpfverdampfer
- W-i2: Kondensator
- W-i3: Sumpfverdampfer
- W-i4: Kondensator

## Patentansprüche

1. Verfahren zur Herstellung von Ethyl-tert.-Butylether (ETBE) aus technischen Mischungen von C₄-Kohlenwasserstoffen I, die mindestens 1-Buten, Isobuten, n-Butan und 2-Butene enthalten, **gekennzeichnet durch** die Verfahrensschritte
a) Umsetzung von Teilen des in der technischen Mischung enthaltenen Isobutens mit Ethanol in Gegenwart eines sauren Katalysators zu ETBE,
b) Abtrennung der nicht umgesetzten C₄-Kohlenwasserstoffe III aus dem Austrag der Stufe a) **durch** thermische Trennverfahren unter Erhalt einer ETBE aufweisenden Fraktion II,
c) destillative Trennung der C₄-Kohlenwasserstoffe III in eine mindestens 1-Buten und Isobuten enthaltende Fraktion IV und eine nahezu Isobuten-freie, mindestens 2-Butene und n-Butan enthaltende Fraktion V,
d) erneute Umsetzung des in Fraktion IV enthaltenen Isobutens mit Ethanol VI in Gegenwart eines sauren Katalysators zu ETBE,
e) Abtrennung der nicht umgesetzten C₄-Kohlenwasserstoffe VIII aus dem Austrag von Stufe d) unter Erhalt einer ETBE aufweisenden Fraktion VII, wobei die in Schritt e) erhaltene C₄-Kohlenwasserstofffraktion VIII **durch** Destillation in einer oder mehreren Destillationskolonnen zu einem 1-Buten, welches einen Gehalt an Isobuten von kleiner 2000 wppm aufweist, aufgearbeitet wird.

2. Verfahren nach Anspruch 1,
**dadurch gekennzeichnet,**
**dass** die sauer katalysierte Veretherung in Stufe d) so durchgeführt wird, dass zumindest eine Reaktionsstufe als Reaktivdestillation durchgeführt wird.

3. Verfahren nach Anspruch 1 oder 2,
**dadurch gekennzeichnet, dass**
dass die sauer katalysierte Veretherung in Stufe d) in mindestens zwei Reaktionsstufen durchgeführt wird, wobei mindestens die letzte Reaktionsstufe als Reaktivdestillation durchgeführt wird.

4. Verfahren nach zumindest einem der Ansprüche 1 bis 3,
**dadurch gekennzeichnet,**
**dass** in Stufe d) die letzte Reaktionsstufe als Reaktivdestillation durchgeführt wird, in der die Stufe e) durchgeführt wird.

5. Verfahren nach zumindest einem der Ansprüche 1 bis 4,
**dadurch gekennzeichnet,**
**dass** anschließend an Stufe e) Restmengen an Ethanol in den C₄-Kohlenwasserstoffen in einem Extraktionsschritt mit Wasser ausgewaschen werden.

6. Verfahren nach zumindest einem der Ansprüche 1 bis 5,
**dadurch gekennzeichnet,**
**dass** die sauer katalysierte Veretherung in Stufe a) so durchgeführt wird, dass zumindest eine Reaktionsstufe als Reaktivdestillation durchgeführt wird.

7. Verfahren nach Anspruch 6,
**dadurch gekennzeichnet, dass**
dass die sauer katalysierte Veretherung in Stufe a) in mindestens zwei Reaktionsstufen durchgeführt wird, wobei mindestens die letzte Reaktionsstufe als Reaktivdestillation durchgeführt wird.

8. Verfahren nach Anspruch 6 oder 7,
**dadurch gekennzeichnet,**
**dass** in Stufe a) die letzte Reaktionsstufe als Reaktivdestillation durchgeführt wird, in der die Stufe b) durchgeführt wird.

9. Verfahren nach zumindest einem der Ansprüche 1 bis 8,
**dadurch gekennzeichnet,**
**dass** es zwischen den Stufen b) und c) einen Verfahrensschritt f) aufweist, in dem aus den als Kopfprodukt erhaltenen C₄-Kohlenwasserstoffen III Restmengen an Ethanol in einem Extraktionsschritt mit Wasser ausgewaschen werden.

10. Verfahren nach zumindest einem der Ansprüche 1 bis 9,
**dadurch gekennzeichnet,**
**dass** in den Verfahrensstufen a) und/oder d) zumindest ein Reaktor in Schlaufenfahrweise betrieben wird.

11. Verfahren nach zumindest einem der Ansprüche 1 bis 10,
**dadurch gekennzeichnet,**
**dass** in den C₄-Kohlenwasserstoffströmen enthaltene mehrfach ungesättigten Kohlenwasserstoffe in einer zusätzlichen Reinigungsstufe, die einer oder mehreren der Verfahrensstufen a), b), c) oder d) vorgeschaltet wird, katalytisch hydriert werden.

12. Verfahren nach Anspruch 11,
**dadurch gekennzeichnet,**
**dass** die Hydrierung der mehrfach ungesättigten Verbindungen in mindestens zwei Reaktionsstufen erfolgt, wobei mindestens die letzte Reaktionsstufe in Anwesenheit von 0,05 bis 100 wppm CO durchgeführt wird.

13. Verfahren nach Anspruch 11 oder 12,
**dadurch gekennzeichnet,**
**dass** die Hydrierung als zusätzliche Reinigungsstufe zwischen den Verfahrensstufen b) und c) durchgeführt wird.

14. Verfahren nach zumindest einem der Ansprüche 1 bis 13,
**dadurch gekennzeichnet,**
**dass** die Reaktion in Stufe a) so durchgeführt wird, dass der Umsatz des Isobutens in der Verfahrensstufe a) über 70 % beträgt.

15. Verfahren nach zumindest einem der Ansprüche 1 bis 14,
**dadurch gekennzeichnet,**
**dass** bei der Umsetzung von Isobuten mit Ethanol als saurer Katalysator ein Ionentauscherharz eingesetzt wird.

16. Verfahren nach zumindest einem der Ansprüche 1 bis 15,
**dadurch gekennzeichnet,**
**dass** der in Verfahrensschritt e) bei der Abtrennung der C₄-Kohlenwasserstoffe VIII erhaltene tert.-Butylether und gegebenenfalls nicht umgesetzten Ethanol enthaltende Strom VII ganz oder teilweise in Schritt a) und/oder b) zurückgeführt werden.

17. Verfahren nach zumindest einem der Ansprüche 1 bis 16,
**dadurch gekennzeichnet,**
**dass** in den Verfahrensstufen a) und/oder d) Ethanol eingesetzt wird, der von 0,05 bis 1 Massen-% ETBE als Vergällungsmittel enthält.

## Claims

1. Process for preparing ethyl tert-butyl ether (ETBE) from technical mixtures of C₄ hydrocarbons I which comprise at least 1-butene, isobutene, n-butane and 2-butenes, **characterized by** the process steps of
a) reacting portions of the isobutene present in the technical mixture with ethanol in the presence of an acidic catalyst to give ETBE,
b) removing the unconverted C₄ hydrocarbons III from the effluent of stage a) by a thermal separating process to obtain a fraction II comprising ETBE,
c) distillatively separating the C₄ hydrocarbons III into a fraction IV comprising at least 1-butene and isobutene, and a virtually isobutene-free fraction V comprising at least 2-butenes and n-butane,
d) again reacting the isobutene present in fraction IV with ethanol VI in the presence of an acidic catalyst to give ETBE,
e) removing the unconverted C₄ hydrocarbons VIII from the effluent of stage d) to obtain a fraction VII comprising ETBE, **characterized in that** the C₄ hydrocarbon fraction VIII obtained in step e) being worked up by distillation in one or more distillation columns to give 1-butene which has a content of isobutene of less than 2000 ppmw.

2. Process according to Claim 1,
**characterized in that**
the acid-catalyzed etherification in stage d) is carried out in such a way that at least one reaction stage is carried out as a reactive distillation.

3. Process according to either of Claims 1 and 2,
**characterized in that**
the acid-catalyzed etherification in stage d) is carried out in at least two reaction stages, at least the last reaction stage being carried out as a reactive distillation.

4. Process according to at least one of Claims 1 to 3,
**characterized in that**,
in stage d), the last reaction stage is carried out as a reactive distillation in which stage e) is carried out.

5. Process according to at least one of Claims 1 to 4,
**characterized in that**,
after stage e), residual amounts of ethanol in the C₄ hydrocarbons are scrubbed out in an extraction step with water.

6. Process according to at least one of Claims 1 to 5,
**characterized in that**,
the acid-catalyzed etherification in stage a) is carried out in such a way that at least one reaction stage is carried out as a reactive distillation.

7. Process according to Claim 6,
**characterized in that**
the acid-catalyzed etherification in stage a) is carried out in at least two reaction stages, at least the last reaction stage being carried out as a reactive distillation.

8. Process according to either of Claims 6 and 7,
**characterized in that**,
in stage a), the last reaction stage is carried out as a reactive distillation in which stage b) is carried out.

9. Process according to at least one of Claims 1 to 8,
**characterized in that**
it has, between stages b) and c), a process step f) in which residual amounts of ethanol are scrubbed out of the C₄ hydrocarbons III obtained as the top product in an extraction step with water.

10. Process according to at least one of Claims 1 to 9,
**characterized in that**,
in process stages a) and/or d), at least one reactor is operated in loop mode.

11. Process according to at least one of Claims 1 to 10,
**characterized in that**
polyunsaturated hydrocarbons present in the C₄ hydrocarbon streams are hydrogenated catalytically in an additional purification stage which is connected upstream of one or more of process stages a), b), c) or d).

12. Process according to Claim 11,
**characterized in that**,
the polyunsaturated compounds are hydrogenated in at least two reaction stages, at least the last reaction stage being carried out in the presence of from 0.05 to 100 ppm of CO.

13. Process according to either of Claims 11 and 12,
**characterized in that**
the hydrogenation is carried out as an additional purification stage between process stages b) and c).

14. Process according to at least one of Claims 1 to 13,
**characterized in that**
the reaction in stage a) is carried out in such a way that the conversion of the isobutene in process stage a) is over 70%.

15. Process according to at least one of Claims 1 to 14,
**characterized in that**
the acidic catalyst used in the reaction of isobutene with ethanol is an ion exchange resin.

16. Process according to at least one of Claims 1 to 15,
**characterized in that**
the tert-butyl ether obtained in process step e) in the removal of the C₄ hydrocarbons VIII and stream VII optionally containing unreacted ethanol are recycled fully or partly into step a) and/or b).

17. Process according to at least one of Claims 1 to 16,
**characterized in that**
ethanol which contains from 0.05 to 1% by mass of ETBE as a denaturing agent is used in process stages a) and/or d).

## Revendications

1. Procédé de fabrication d'éther d'éthyle et de tert.-butyle (ETBE) à partir de mélanges techniques d'hydrocarbures en C₄ I, qui contiennent au moins du 1-butène, de l'isobutène, du n-butane et des 2-butènes, **caractérisé par** les étapes de procédé suivantes :
a) la mise en réaction de parties de l'isobutène contenu dans le mélange technique avec de l'éthanol en présence d'un catalyseur acide pour former de l'ETBE,
b) la séparation des hydrocarbures en C₄ III non réagis de la sortie de l'étape a) par des procédés de séparation thermiques pour obtenir une fraction II comprenant de l'ETBE,
c) la séparation distillative des hydrocarbures en C₄ III en une fraction IV contenant au moins du 1-butène et de l'isobutène et une fraction V pratiquement exempte d'isobutène, contenant au moins des 2-butènes et du n-butane,
d) la nouvelle mise en réaction de l'isobutène contenu dans la fraction IV avec de l'éthanol VI en présence d'un catalyseur acide pour former de l'ETBE,
e) la séparation des hydrocarbures en C₄ VIII non réagis de la sortie de l'étape d) pour obtenir une fraction VII comprenant de l'ETBE, la fraction d'hydrocarbures en C₄ VIII obtenue à l'étape e) étant traitée par distillation dans une ou plusieurs colonnes de distillation pour former un 1-butène, qui présente une teneur en isobutène inférieure à 2 000 ppm en poids.

2. Procédé selon la revendication 1, **caractérisé en ce que** l'éthérification sous catalyse acide à l'étape d) est réalisée de manière à ce qu'au moins une étape de réaction soit réalisée sous la forme d'une distillation réactive.

3. Procédé selon la revendication 1 ou 2, **caractérisé en ce que** l'éthérification sous catalyse acide à l'étape d) est réalisée en au moins deux étapes de réaction, au moins la dernière étape de réaction étant réalisée sous la forme d'une distillation réactive.

4. Procédé selon au moins l'une quelconque des revendications 1 à 3, **caractérisé en ce que** la dernière étape de réaction à l'étape d) est réalisée sous la forme d'une distillation réactive, lors de laquelle l'étape e) est réalisée.

5. Procédé selon au moins l'une quelconque des revendications 1 à 4, **caractérisé en ce qu'**après l'étape e), des quantités résiduelles d'éthanol dans les hydrocarbures en C₄ sont éliminées par rinçage avec de l'eau lors d'une étape d'extraction.

6. Procédé selon au moins l'une quelconque des revendications 1 à 5, **caractérisé en ce que** l'éthérification sous catalyse acide à l'étape a) est réalisée de manière à ce qu'au moins une étape de réaction soit réalisée sous la forme d'une distillation réactive.

7. Procédé selon la revendication 6, **caractérisé en ce que** l'éthérification sous catalyse acide à l'étape a) est réalisée en au moins deux étapes de réaction, au moins la dernière étape de réaction étant réalisée sous la forme d'une distillation réactive.

8. Procédé selon la revendication 6 ou 7, **caractérisé en ce que** la dernière étape de réaction à l'étape a) est réalisée sous la forme d'une distillation réactive, lors de laquelle l'étape b) est réalisée.

9. Procédé selon au moins l'une quelconque des revendications 1 à 8, **caractérisé en ce qu'**il comprend entre les étapes b) et c) une étape de procédé f) lors de laquelle des quantités résiduelles d'éthanol dans les hydrocarbures en C₄ III obtenus en tant que produit de tête sont éliminées par rinçage avec de l'eau lors d'une étape d'extraction.

10. Procédé selon au moins l'une quelconque des revendications 1 à 9, **caractérisé en ce qu'**au moins un réacteur est exploité en mode à boucle dans les étapes de procédé a) et/ou d) .

11. Procédé selon au moins l'une quelconque des revendications 1 à 10, **caractérisé en ce que** les hydrocarbures polyinsaturés contenus dans les courants d'hydrocarbures en C₄ sont hydrogénés catalytiquement lors d'une étape de purification supplémentaire qui précède une ou plusieurs des étapes de procédé a), b), c) ou d).

12. Procédé selon la revendication 11, **caractérisé en ce que** l'hydrogénation des composés polyinsaturés a lieu en au moins deux étapes de réaction, au moins la dernière étape de réaction étant réalisée en présence de 0,05 à 100 ppm en poids de CO.

13. Procédé selon la revendication 11 ou 12, **caractérisé en ce que** l'hydrogénation est réalisée sous la forme d'une étape de purification supplémentaire entre les étapes de procédé b) et c).

14. Procédé selon au moins l'une quelconque des revendications 1 à 13, **caractérisé en ce que** la réaction à l'étape a) est réalisée de manière à ce que la conversion de l'isobutène dans l'étape de procédé a) soit supérieure à 70 %.

15. Procédé selon au moins l'une quelconque des revendications 1 à 14, **caractérisé en ce qu'**une résine échangeuse d'ions est utilisée en tant que catalyseur acide lors de la réaction de l'isobutène avec l'éthanol.

16. Procédé selon au moins l'une quelconque des revendications 1 à 15, **caractérisé en ce que** le courant VII contenant de l'éther de tert.-butyle et éventuellement de l'éthanol non réagi obtenu à l'étape de procédé e) lors de la séparation des hydrocarbures en C₄ VIII est recyclé en totalité ou en partie dans l'étape a) et/ou b).

17. Procédé selon au moins l'une quelconque des revendications 1 à 16, **caractérisé en ce que** de l'éthanol qui contient 0,05 à 1% en masse d'ETBE en tant qu'agent de dénaturation est utilisé dans les étapes de procédé a) et /ou d).
